# EUROPEAN PATENT SPECIFICATION

(11) **EP 4 556 963 B1**
(45) Date of publication and mention of the grant of the patent: **25.02.2026**
(21) Application number: 23893281.8
(22) Date of filing: 14.08.2023
(51) Int. Cl.: G02B 3/00, A61B 5/024

(54) **OPTICAL LENS, PHOTOPLETHYSMOGRAPHY DEVICE, AND ELECTRONIC DEVICE**
OPTISCHE LINSE, PHOTOPLETHYSMOGRAFIEVORRICHTUNG UND ELEKTRONISCHE VORRICHTUNG
LENTILLE OPTIQUE, DISPOSITIF DE PHOTOPLÉTHYSMOGRAPHIE ET DISPOSITIF ÉLECTRONIQUE

(30) Priority: 26.11.2022 CN 202211494769
(43) Date of publication of application: 21.05.2025
(73) Proprietor: Honor Device Co., Ltd., Shenzhen, Guangdong 518040 (CN)
(72) Inventor: SHANG, Ruiying, Shenzhen, Guangdong 518040 (CN); SU, Dan, Shenzhen, Guangdong 518040 (CN); TAN, Yinjiong, Shenzhen, Guangdong 518040 (CN); LIU, Yi, Shenzhen, Guangdong 518040 (CN)
(74) Representative: Gill Jennings & Every LLP
(86) International application number: PCT/CN2023/112928
(87) International publication number: WO 2024/109182

(56) References cited:
- WO-A1-2019/062480
- WO-A1-2021/042395
- CN-A- 109 557 666
- CN-A- 111 095 283
- CN-U- 215 297 723
- JP-A- 2005 055 485
- JP-A- 2009 223 192
- US-A1- 2014 055 864
- US-A1- 2014 327 763
- US-A1- 2014 327 763
- US-A1- 2020 367 967

## Description

### TECHNICAL FIELD

This application relates to the technical field of electronic devices, and in particular, to an optical lens, a photoplethysmograph, and an electronic device.

### BACKGROUND

There is a light emitter in devices such as a photoplethysmograph, a projector, a camera device, and a camera, the light emitter is used to emit light beams to realize functions of heart rate detection, illumination, light supplement, and the like. In addition, a light emitting side of the light emitter is usually provided with a light-transmitting cover plate to protect the light emitter from water, dust, and scratches without blocking an optical path.

Due to different refractive indexes of the light-transmitting cover plate and air, light may be reflected on surfaces of the light-transmitting cover plate that respectively face the light emitter and face away from the light emitter. As a result, light emitting efficiency is reduced, and emitted light energy is less. Moreover, light emitted from the light-transmitting cover plate has a large light emitting angle. This leads to a low maximum intensity of light emitted from the light-transmitting cover plate, thereby affecting user experience. Relevant prior art is disclosed in WO 2021/042395 A1, WO 2019/062480 A1, US 2014/327763 A1, CN 215 297 723 U, US 2014/055864 A1, CN 111 095 283 A, CN 109 557 666 A, US 2020/367967 A1, JP 2009 223192 A, JP 2005 055485 A, US 2014/327763 A1.

### SUMMARY

Embodiments of this application provide an optical lens, a photoplethysmograph, and an electronic device, to solve the problem of how to reduce reflectivity of light at a cover plate, reduce a light emitting angle of light emitted from the cover plate, and increase light emitting energy and a maximum intensity of light.

To achieve the foregoing objective, the following technical solutions are used in the embodiments of this application.

According to a first aspect, an optical lens is provided, where the optical lens includes a light-transmitting substrate and a microlens array, and the light-transmitting substrate is provided with a first surface and a second surface opposite to each other. The microlens array is arranged on the first surface, and the microlens array includes a first microlens assembly and a plurality of second microlens assembly arrays. Each of the second microlens assembly arrays includes a plurality of second microlens assemblies arranged around the first microlens assembly in an array, and the plurality of second microlens assembly arrays are sequentially arranged along a radial direction of an annular array track of the plurality of second microlens assemblies. Both the first microlens assembly and the second microlens assembly include a first microlens unit and a second microlens array, the first microlens unit includes at least one first microlens, and the second microlens array includes a plurality of second microlenses arranged around the first microlens unit in an array. Both the first microlens and the second microlens are convex spheres protruding from the first surface in a direction away from the second surface.

In this way, when the optical lens is used between the light emitter and the cover plate, the first surface of the optical lens faces the light emitter, and the second surface faces the cover plate, light beams emitted by the light emitter may be converged by using the first microlens and the second microlens, to reduce incident angles of some light on surfaces of the cover plate that respectively face the light emitter and face away from the light emitter. Therefore, an amount of light reflected by the cover plate can be reduced. Moreover, light beams emitted by the light emitter are converged by using the first microlens and the second microlens, and a light emitting angle of light emitted from the cover plate can be reduced. In addition, some gaps are formed between two adjacent second microlens assembly arrays. These gaps are not provided with convex spheres, which can reduce an energy loss of light when passing through a microlens array. Therefore, it is beneficial to improve light emitting efficiency, and increase light emitting energy and a maximum intensity of light. In addition, the microlens array occupies a small height, which is convenient for installation in an electronic device with limited space.

In a possible implementation of the first aspect, two adjacent second microlens assembly arrays are arranged at intervals and closely along the radial direction of the annular array track of the plurality of second microlens assemblies, and the first microlens assembly and the adjacent second microlens assembly array are arranged at intervals and closely along the radial direction of the annular array track of the plurality of second microlens assemblies. In this way, a quantity of second microlens assembly arrays arranged on the first surface is large, light beams emitted by the light emitter can be converged as much as possible, and incident angles of some light on surfaces of the cover plate that respectively face the light emitter and face away from the light emitter are reduced. Therefore, an amount of light reflected by the cover plate can be reduced. Moreover, light beams emitted by the light emitter can be converged as much as possible, and a light emitting angle of light emitted from the cover plate can be reduced. Therefore, light emitting efficiency can be improved, and light emitting energy and a maximum intensity of light can be increased.

In a possible implementation of the first aspect, a plurality of second microlens assemblies in each second microlens assembly array are also arranged at intervals and closely along an annular array track of the plurality of second microlens assemblies. In this way, a quantity of second microlens assemblies arranged in each second microlens assembly array is large, light beams emitted by the light emitter can be converged as much as possible, and incident angles of some light on surfaces of the cover plate that respectively face the light emitter and face away from the light emitter are reduced. Therefore, an amount of light reflected by the cover plate can be reduced. Moreover, light beams emitted by the light emitter can be converged as much as possible, and a light emitting angle of light emitted from the cover plate can be reduced. Therefore, light emitting efficiency can be improved, and light emitting energy and a maximum intensity of light can be increased.

In a possible implementation of the first aspect, the second microlens array and the first microlens unit are arranged at intervals and closely along a radial direction of an annular array track of the plurality of second microlenses. In this way, an occupied area of the first microlens assembly and the second microlens assembly on the first surface is small, and a quantity of first microlens assemblies and second microlens assemblies that can be arranged on the first surface is large. Light beams emitted by the light emitter can be converged as much as possible, and incident angles of some light on surfaces of the cover plate that respectively face the light emitter and face away from the light emitter are reduced. Therefore, an amount of light reflected by the cover plate can be reduced. Moreover, light beams emitted by the light emitter can be converged as much as possible, and a light emitting angle of light emitted from the cover plate can be reduced. Therefore, light emitting efficiency can be improved, and light emitting energy and a maximum intensity of light can be increased.

In a possible implementation of the first aspect, in the second microlens array, a plurality of second microlenses are also arranged at intervals and closely along an annular array track of the plurality of second microlenses. In this way, a quantity of second microlenses arranged in the second microlens array is large, light beams emitted by the light emitter can be converged as much as possible, and incident angles of some light on surfaces of the cover plate that respectively face the light emitter and face away from the light emitter are reduced. Therefore, an amount of light reflected by the cover plate can be reduced. Moreover, light beams emitted by the light emitter can be converged as much as possible, and a light emitting angle of light emitted from the cover plate can be reduced. Therefore, light emitting efficiency can be improved, light energy and a maximum intensity of light entering the human body are increased, and measurement accuracy and a perfusion index are improved.

In a possible implementation of the first aspect, a quantity of the first microlenses in the first microlens unit is one, and a quantity of the second microlenses in the second microlens array is six.

In a possible implementation of the first aspect, there are a plurality of the first microlenses in the first microlens unit, the plurality of first microlenses are arranged in a row, and an arrangement direction of the plurality of first microlenses in the second microlens assembly is consistent with an extension direction of an annular array track of a second microlens assembly array in which the second microlens assembly is located. In this way, it is convenient for a plurality of first microlenses to be positioned in the first surface, which is conducive to reducing design difficulty.

In a possible implementation of the first aspect, in the first microlens assembly and the second microlens assembly, the plurality of first microlenses are arranged at intervals and closely. That the plurality of first microlenses are arranged at intervals indicates: in the plurality of first microlenses, a spacing between two adjacent first microlenses is greater than or equal to 0. In addition, that the plurality of first microlenses are arranged closely indicates: a spacing between two adjacent first microlenses is less than or equal to 0.003 millimeters. In this way, an occupied area of the first microlens unit on the first surface is small, which is conducive to reducing the occupied area of the first microlens assembly and the second microlens assembly on the first surface, so that a quantity of first microlens assemblies and second microlens assemblies that can be arranged on the first surface is large. Light beams emitted by the light emitter can be converged as much as possible, and incident angles of some light on surfaces of the cover plate that respectively face the light emitter and face away from the light emitter are reduced. Therefore, an amount of light reflected by the cover plate can be reduced. Moreover, light beams emitted by the light emitter can be converged as much as possible, and a light emitting angle of light emitted from the cover plate can be reduced. Therefore, light emitting efficiency can be improved, and light energy and a maximum intensity of light entering the human body are increased.

In a possible implementation of the first aspect, in both the first microlens assembly and the second microlens assembly, a quantity of the first microlenses is two, and a quantity of the second microlenses in the second microlens array is twelve.

In a possible implementation of the first aspect, in both the first microlens assembly and the second microlens assembly, a quantity of the first microlenses is two, and a quantity of the second microlenses in the second microlens array is six.

In a possible implementation of the first aspect, a diameter of an occupied region of the first microlens on the first surface is greater than or equal to 0.1 mm and less than or equal to 0.15 mm, a curvature of an end that is of the first microlens and that is away from the first surface is 0 mm⁻¹, a curvature of an end that is of the first microlens and that is connected to the first surface is greater than or equal to 8 mm⁻¹ and less than or equal to 10 mm⁻¹, and a height of the first microlens protruding from the first surface is greater than or equal to 0.0252 mm and less than or equal to 0.034 mm. In this way, the first microlens can converge light beams better and can reduce incident angles of some light on surfaces of the cover plate that respectively face the light emitter and face away from the light emitter. Therefore, an amount of light reflected by the cover plate can be reduced. Moreover, the first microlens can converge light beams better, and can further reduce a light emitting angle of light emitted from the cover plate. Therefore, light energy and a maximum intensity of light entering the human body are increased, and measurement accuracy and a perfusion index are improved.

In a possible implementation of the first aspect, a diameter of an occupied region of the second microlens on the first surface is greater than or equal to 0.05 mm and less than or equal to 0.1 mm, a curvature of an end that is of the second microlens and that is away from the first surface is 0 mm⁻¹, a curvature of an end that is of the second microlens and that is connected to the first surface is greater than or equal to 5 mm⁻¹ and less than or equal to 40 mm⁻¹, and a height of the second microlens protruding from the first surface is greater than or equal to 0.01 mm and less than or equal to 0.0175 mm. In this way, the second microlens can converge light beams better and can reduce incident angles of some light on surfaces of the cover plate that respectively face the light emitter and face away from the light emitter. Therefore, an amount of light reflected by the cover plate can be reduced. Moreover, the second microlens can converge light beams better, and can further reduce a light emitting angle of light emitted from the cover plate. Therefore, light energy and a maximum intensity of light entering the human body are increased, and measurement accuracy and a perfusion index are improved.

In a possible implementation of the first aspect, the microlens array is integrally formed with the light-transmitting substrate, and material of the microlens array and the light-transmitting substrate includes at least one of glass, sapphire, polyamide, polycarbonate, polymeric methyl methacrylate, thermoplastic polyurethanes, and transparent nylon.

According to a second aspect, a photoplethysmograph is provided, where the photoplethysmograph includes a cover plate, a light emitter, a light receiver, and an optical lens. The cover plate includes a first light transmissive part and a second light transmissive part. The light emitter is located on an inner side of the cover plate, and a light emitting surface of the light emitter faces the first light transmissive part. The light receiver is located on the inner side of the cover plate, and a light incident surface of the light receiver faces the second light transmissive part. The optical lens is the optical lens according to any one of the foregoing technical solutions, the optical lens is disposed on a surface that is of the first light transmissive part and that faces the light emitter, a first surface of the optical lens faces the light emitter, and a second surface faces the first light transmissive part.

In this way, light beams emitted by the light emitter may be converged by using the first microlens and the second microlens, to reduce incident angles of some light on surfaces of the cover plate that respectively face the light emitter and face away from the light emitter. Therefore, an amount of light reflected by the cover plate can be reduced. Moreover, light beams emitted by the light emitter are converged by using the first microlens and the second microlens, and a light emitting angle of light emitted from the cover plate can be reduced. In addition, some gaps are formed between two adjacent second microlens assembly arrays. These gaps are not provided with convex spheres, which can reduce an energy loss of light when passing through a microlens array. Therefore, it is beneficial to improve light emitting efficiency, increase light energy and a maximum intensity of light entering the human body, and improve measurement accuracy and a perfusion index.

In a possible implementation of the second aspect, an array center of a microlens array of the optical lens is aligned with the light emitting surface of the light emitter. In this way, light emitted by the light emitter can be converged better, an amount of light that is emitted by the light emitter and that is reflected by the cover plate can be reduced, and a light emitting angle of light emitted from the cover plate can be reduced.

In a possible implementation of the second aspect, the optical lens is integrally formed with the first light transmissive part. In this way, there is no other medium between the optical lens and the first light transmissive part, which can ensure light emitting efficiency, and isolate mixing light, to avoid light from leaking between the optical lens and the first light transmissive part. In addition, there is no need for an attaching operation, thereby reducing process difficulty.

In a possible implementation of the second aspect, the cover plate further includes a light shielding part, the light shielding part is opaque to light, and the light shielding part is located between at least the first light transmissive part and the second light transmissive part. In this way, the light shielding part is used to prevent, as much as possible, some light emitted by the light emitter from directly entering a collection range of the light receiver without passing through a human body part to be detected, to avoid affecting detection accuracy of the photoplethysmograph.

According to a third aspect, an electronic device is provided, where the electronic device includes a display, a casing, and the photoplethysmograph according to any one of the foregoing technical solutions. The display is connected to the casing, the casing includes a back cover, and the back cover is opposite to the display. A cover plate of the photoplethysmograph is disposed on the back cover, and a light emitter and a light receiver of the photoplethysmograph are located in the casing.

Because the electronic device provided in this application includes the photoplethysmograph according to any one of the technical solutions in the second aspect, the electronic device and the photoplethysmograph can solve the same technical problem and achieve the same effect.

### BRIEF DESCRIPTION OF DRAWINGS

FIG. 1 is a schematic diagram of a structure of an electronic device according to some embodiments of this application;
FIG. 2 is an exploded diagram of a watch body in an electronic device shown in FIG. 1;
FIG. 3 is a schematic diagram of a cross-sectional structure of an electronic device shown in FIG. 1 at a line C-C;
FIG. 4 is an enlarged diagram of an electronic device shown in FIG. 3 at region I;
FIG. 5 is a bottom view of a light emitter and a light receiver in an electronic device shown in FIG. 4;
FIG. 6 is a top view of a cover plate in an electronic device shown in FIG. 4;
FIG. 7 is a schematic diagram of a structure of an electronic device shown in FIG. 4 in a case of being worn on a wrist of a user;
FIG. 8 is an enlarged diagram of an optical lens in an electronic device shown in FIG. 1-FIG. 4;
FIG. 9 is a schematic diagram of a structure of an optical lens shown in FIG. 8 on a first surface;
FIG. 10 is a schematic diagram of a structure of a first microlens assembly or a second microlens assembly in a microlens array in an optical lens shown in FIG. 9;
FIG. 11 is a schematic diagram of a structure of a second microlens assembly array in an optical lens shown in FIG. 9;
FIG. 12 is a schematic diagram of a structure of two adjacent second microlens assembly arrays in an optical lens shown in FIG. 9;
FIG. 13 is a schematic diagram of a structure of a new array formed by arranging remaining second microlens assemblies along an annular array after a second microlens assembly array located at an outer layer in two adjacent second microlens assembly arrays shown in FIG. 12 is reduced by one second microlens assembly;
FIG. 14 is a schematic diagram of a structure of a second microlens assembly array in an optical lens shown in FIG. 9;
FIG. 15 is a schematic diagram of a structure of an optical lens on a first surface according to some other embodiments of this application;
FIG. 16 is a schematic diagram of a structure of a first microlens assembly or a second microlens assembly in a microlens array in an optical lens shown in FIG. 15;
FIG. 17 is a schematic diagram of a structure of an optical lens on a first surface according to some other embodiments of this application;
FIG. 18 is a schematic diagram of a structure of a first microlens assembly or a second microlens assembly in a microlens array in an optical lens shown in FIG. 17;
FIG. 19 is a side view of a structure including a single first microlens and a light-transmitting substrate in a first microlens assembly or a second microlens assembly shown in FIG. 10, FIG. 16, or FIG. 18;
FIG. 20 is a side view of a structure including a single second microlens and a light-transmitting substrate in a first microlens assembly or a second microlens assembly shown in FIG. 10, FIG. 16, or FIG. 18;
FIG. 21 is a schematic diagram of a structure of a photoplethysmograph in an electronic device according to some other embodiments of this application; and
FIG. 22 is a structural block diagram of a circuit board assembly in an electronic device according to some embodiments of this application.

### DESCRIPTION OF EMBODIMENTS

In the embodiments of this application, terms "first" and "second" are used merely for the purpose of description, and shall not be construed as indicating or implying relative importance or implying a quantity of indicated technical features. Therefore, a feature limited by "first" or "second" may explicitly or implicitly include one or more of the features.

In the embodiments of this application, the term "include", "comprise", or any other variant thereof is intended to cover a non-exclusive inclusion, so that a process, a method, an article, or an apparatus that includes a list of elements not only includes those elements but also includes other elements which are not expressly listed, or further includes elements inherent to such process, method, article, or apparatus. In absence of more constraints, an element preceded by a statement "includes a..." does not preclude the presence of additional identical elements in the process, method, article, or apparatus that includes the element.

"And/or" in the embodiments of this application describes only an association relationship for describing associated objects and indicates that three relationships may exist. For example, A and/or B may represent the following three cases: Only A exists, both A and B exist, and only B exists. In addition, the character "/" in this specification generally indicates an "or" relationship between the associated objects.

This application provides an optical lens, and the optical lens may be used in a photoplethysmograph. For ease of understanding the photoplethysmograph provided in the embodiments of this application, the following first describes application scenarios of the photoplethysmograph. The photoplethysmograph may be used in electronic devices, for example, common electronic products such as a mobile phone, a tablet computer, a digital camera, a wearable device, or large and small medical devices that measure physiological parameters (heart rate, blood oxygen, blood pressure, blood glucose, and the like) by using an optical method. The wearable device includes but is not limited to a smart watch, a smart bracelet, and smart clothes.

The photoplethysmograph is to detect a heart rate change of a tested user by using a photoplethysmograph (photoplethysmograph, PPG) technology. The PPG technology refers to a non-invasive detection technology for detecting blood volume changes in living tissues with optoelectronic means.

The photoplethysmograph includes a light emitter and a light receiver. When the light emitter irradiates a light beam with a specific wavelength to a skin surface of a tested user, the light beam may be transmitted to the light receiver through reflection. In this process, because a volume of blood in blood vessels changes in a fluctuating manner with cardiac contraction and relaxation, when the heart contracts, a blood volume of peripheral blood vessels of the heart increases, light absorption also increases, and then a light intensity detected by the light receiver is low; and when the heart relaxes, the blood volume of the peripheral blood vessels of the heart decreases, and the light intensity detected by the light receiver is great. It can be learned that the light intensity detected by the light receiver changes in a pulsating manner, and a heart rate of a wearer may be obtained after the light intensity change signal is converted into a digital electrical signal. Through a heart rate signal, information of physiological parameters such as blood pressure, blood oxygen, brain oxygen, muscle oxygen, blood glucose, pulse rate, and respiratory rate of a tested user may be further obtained. Therefore, at present, the electronic device such as a smart watch and a smart bracelet uses the photoplethysmograph to track a state of health of the user.

Generally, the light emitter and the light receiver are disposed adjacent to each other in the electronic device, and the photoplethysmograph usually further includes a cover plate, where the cover plate is used to protect the light emitter and the light receiver from water, dust, and scratches. Light emitted by the light emitter is easily reflected at the cover plate, this reduces light emitting efficiency, and light energy entering a human body is less. Moreover, light emitted from the cover plate has a large light emitting angle. This leads to a low maximum intensity of light entering the human body, and affects detection accuracy and a perfusion index (perfusion index, PI), thereby affecting user experience.

Therefore, an embodiment of this application provides an improved electronic device.

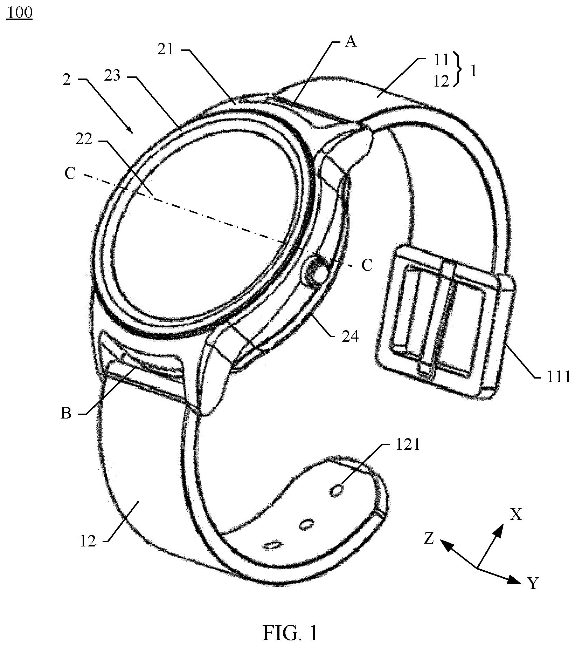

The following describes the electronic device provided in the embodiments of this application with reference to the accompanying drawings. Refer to FIG. 1. FIG. 1 is a schematic diagram of a structure of an electronic device 100 according to some embodiments of this application. In this embodiment and the following embodiments, an example in which the electronic device 100 is a smart watch is used for description, and this cannot be considered as a special limitation to a structural form of the electronic device 100. The smart watch includes a watch body 2 and a watchband 1. The watch body 2 is used to realize a main function of the smart watch, and the watchband 1 is used to wear the watch body 2 on a wrist of a human body.

In some embodiments, still refer to FIG. 1, the watchband 1 may include a first watchband part 11 and a second watchband part 12. One end of the first watchband part 11 and one end of the second watchband part 12 are connected to opposite ends of the watch body 2. The other end of the first watchband part 11 is provided with a first locking portion 111, and the other end of the second watchband part 12 is provided with a second locking portion 121. The first locking portion 111 and the second locking portion 121 are detachably locked to each other, so that the watch body 2 can be worn on a wrist of a user.

A mating structure of the first locking portion 111 and the second locking portion 121 may be a buckle structure such as a hook buckle, a concealed buckle, a butterfly buckle, a belt buckle, a foldable safety buckle, a foldable buckle, a pin buckle, or the like. This is not specifically limited in this application.

The following mainly describes the watch body 2.

Still refer to FIG. 1, the watch body 2 is approximately disc-shaped. On this basis, for ease of description of the following embodiments, an XYZ coordinate system is established for the watch body 2 in this embodiment and the following embodiments. Specifically, a thickness direction of the watch body 2 is defined as a Z-axis direction, and a plane perpendicular to the Z-axis direction is an XY plane; and a position on the watch body 2 for connecting the first watchband part 11 is a first position A, a position on the watch body 2 for connecting the second watchband part 12 is a second position B, and the first position A and the second position B are located in the XY plane. Based on this, an arrangement direction of the first position A and the second position B is defined as an X-axis direction, and a direction perpendicular to the X-axis direction in the XY plane is a Y-axis direction. It can be understood that the coordinate system of the watch body 2 may be flexibly set according to actual requirements. This is not specifically limited herein. In some other embodiments, the watch body 2 may be approximately elliptical disc-shaped, triangular disc-shaped, polygonal disc-shaped, or rectangular disc-shaped. This is not specifically limited herein.

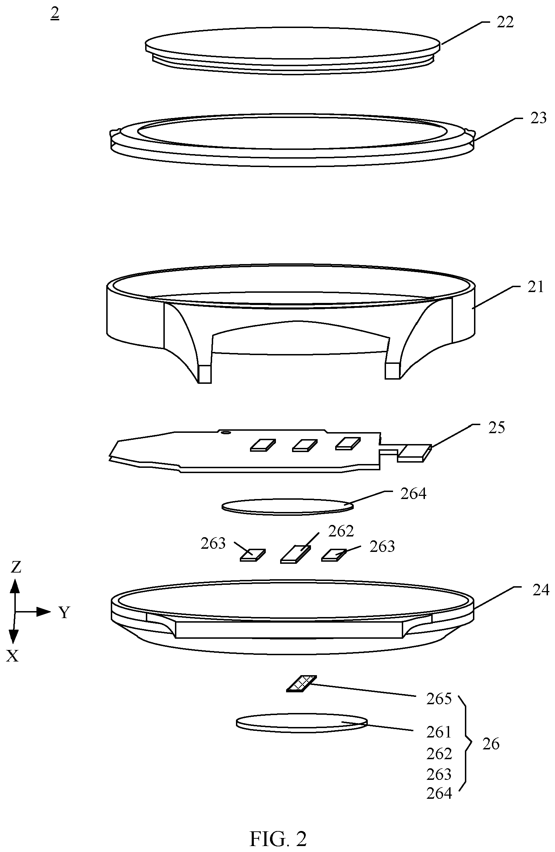

Refer to FIG. 1 and FIG. 2 together. FIG. 2 is an exploded diagram of a watch body 2 in an electronic device 100 shown in FIG. 1. The watch body 2 includes a watch frame 21, a screen 22, an upper casing 23, a lower casing 24, a circuit board assembly 25, a battery (not shown in the figure), and a photoplethysmograph 26.

It can be understood that FIG. 1 and FIG. 2 schematically show some components included in the watch body 2, and actual shapes, actual sizes, actual positions, and actual structures of these components are not limited by FIG. 1 and FIG. 2. In some other embodiments, the watch body 2 may not include the screen 22.

The watch frame 21 is a supporting frame of the watch body 2, and the first position A and the second position B are located on the watch frame 21. Material of the watch frame 21 may be stainless steel to ensure structure strength of the watch frame 21 and support strength for other components.

The upper casing 23 is connected to one end of the watch frame 21 along the Z-axis direction, and the lower casing 24 is connected to the other end of the watch frame 21 along the Z-axis direction. Material of the upper casing 23 and the lower casing 24 includes, but is not limited to, metal material such as stainless steel and plastics such as polycarbonate (polycarbonate, PC), PC+ glass fiber, and ABS plastic (acrylonitrile butadiene styrene plastic).

The upper casing 23, the watch frame 21, and the lower casing 24 form a casing of the watch body 2, and the lower casing 24 forms a back cover of the casing. An accommodating space is formed inside the casing, and the circuit board assembly 25 and the battery are accommodated in the accommodating space. The battery is used to supply power to the circuit board assembly 25.

The screen 22 is fixed to the upper casing 23. The screen 22 is used to display numerical values or graphs such as hour hand, minute hand, second hand, dial, digital time, weather, air temperature, electrocardiogram (electrocardiogram, ECG), body temperature, heart rate, body fat, and voltage.

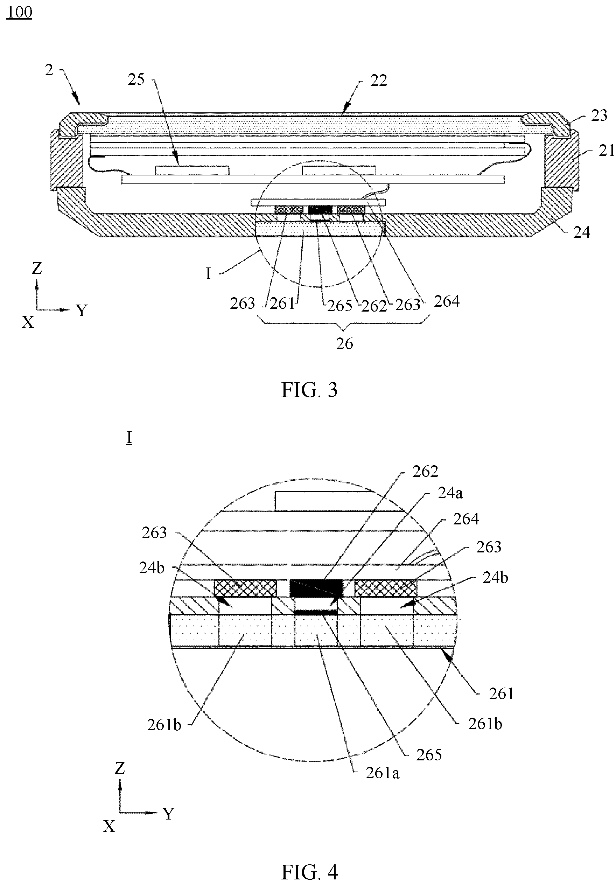

The photoplethysmograph 26 detects a heart rate change of a wearer by using the PPG technology. Refer to FIG. 2 and FIG. 3 together. FIG. 3 is a schematic diagram of a cross-sectional structure of an electronic device 100 shown in FIG. 1 at a line C-C. The photoplethysmograph 26 includes a cover plate 261, a light emitter 262, a light receiver 263, and a circuit board 264.

The cover plate 261 is used to protect the light emitter 262 and the light receiver 263. The cover plate 261 is disposed on the lower casing 24.

In some embodiments, refer to FIG. 4, FIG. 4 is an enlarged diagram of an electronic device 100 shown in FIG. 3 at region I, and the lower casing 24 is provided with a first light hole 24a and a second light hole 24b. The first light hole 24a allows a light beam emitted by the light emitter 262 to pass through and enter an outer side of the lower casing 24, and the second light hole 24b allows the light beam on the outer side of the lower casing 24 to pass through and enter the light receiver 263. The first light hole 24a and the second light hole 24b may be in circular, rectangular, polygonal, or other irregular shapes. It should be noted that, in the foregoing embodiment and the following embodiments, the "outer side" used to describe the "lower casing 24" refers to a side that is of the lower casing 24 and that faces away from the accommodating space, an "outer surface" refers to a surface that is of the lower casing 24 and that faces away from the accommodating space, and the "inner side" refers to a side that is of the lower casing 24 and that faces the accommodating space. This may not be described again in the following.

On the basis of the above, the cover plate 261 is disposed on the outer surface of the lower casing 24 and covers the first light hole 24a and the second light hole 24b.

In this way, a retaining wall is formed with a part of the lower casing 24 between the first light hole 24a and the second light hole 24b, to separate light emitted by the light emitter 262 and light received by the light receiver 263, thereby reducing mixing light between the light emitter 262 and the light receiver 263.

In other embodiments, the lower casing 24 may also be provided with an opening, and the cover plate 261 covers the opening. Alternatively, a part of the lower casing 24 forms the cover plate 261. In this way, there is no thickness addition between the cover plate 261 and the lower casing 24 in the Z-axis direction, which is conducive to reducing a height of the watch body 2 in the Z-axis direction.

Still refer to FIG. 4. The cover plate 261 includes a first light transmissive part 261a and a second light transmissive part 261b. The first light transmissive part 261a covers the first light hole 24a, and the second light transmissive part 261b covers the second light hole 24b. The first light transmissive part 261a allows a light beam emitted by the light emitter 262 to pass through and enter an outer side of the cover plate 261, and the second light transmissive part 261b allows a light beam on the outer side of the cover plate 261 to pass through and enter the light receiver 263. It should be noted that, in the foregoing embodiment and the following embodiments, the "outer side" used to describe the "cover plate 261" refers to a side that is of the cover plate 261 and that faces away from the accommodating space, an "outer surface" refers to a surface that is of the cover plate 261 and that faces away from the accommodating space, and the "inner side" refers to a side that is of the cover plate 261 and that faces the accommodating space. This may not be described again in the following.

The rest part of the cover plate 261 other than the first light transmissive part 261a and the second light transmissive part 261b may be in a light transmissive or opaque structure, or partially in a light transmissive structure and partially in an opaque structure. An embodiment shown in FIG. 4 uses an example in which all of the rest part is in the opaque structure for description, and this cannot be considered as a special limitation to this application.

In the foregoing embodiment, specific material of the cover plate 261 is not limited in this embodiment of this application, as long as light transmissive requirements of the light emitter 262 and the light receiver 263 are satisfied. The material of the cover plate 261 is sapphire or ultra clear glass, and the sapphire and the ultra clear glass have good light transmittance and high strength. In addition to ensuring light transmittance, impact resistance is ensured.

Refer back to FIG. 3. The light emitter 262, the light receiver 263, the circuit board 264, and the optical lens 265 are all located inside the cover plate 261, and the light emitter 262, the light receiver 263, and the circuit board 264 are also located inside the lower casing 24. The circuit board 264 is located on a side that is of the light emitter 262 and the light receiver 263 and that faces away from the cover plate 261, and the light emitter 262 and the light receiver 263 are carried and electrically connected to the circuit board 264. The circuit board 264 is electrically connected to the circuit board assembly 25.

The light emitter 262 has a light emitting surface, and the light emitting surface of the light emitter 262 faces the first light transmissive part 261a. The light receiver 263 has a light incident surface, and the light incident surface of the light receiver 263 faces the second light transmissive part 261b. The light emitter 262 is used to emit light outward through the light emitting surface, and the light receiver 263 is used to receive an external optical signal through the light incident surface.

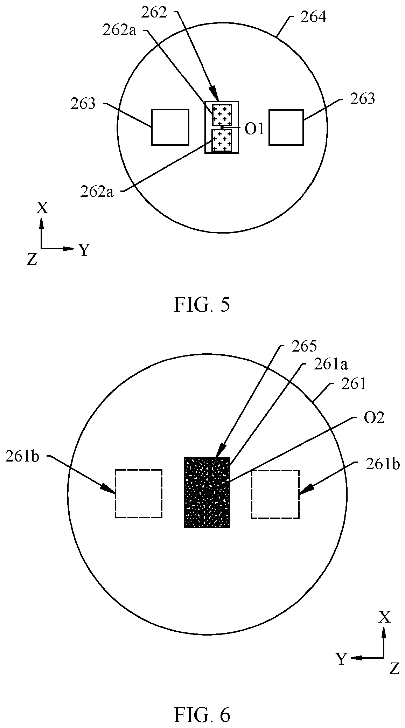

The light emitter 262 may include one lamp bead or a plurality of lamp beads arranged in an array. This is not specifically limited herein. When the light emitter 262 includes one lamp bead, a center O1 of the light emitting surface of the light emitter 262 described below refers to a center of a light emitting surface of the lamp bead; and when the light emitter 262 includes a plurality of lamp beads arranged in an array, the center O1 of the light emitting surface of the light emitter 262 described below refers to an array center of light emitting surfaces of the plurality of lamp beads. For example, refer to FIG. 5, FIG. 5 is a bottom view of a light emitter 262 and a light receiver 263 in an electronic device 100 shown in FIG. 4. The light emitter 262 includes two lamp beads 262a. The center O1 of the light emitting surface of the light emitter 262 is an array center of light emitting surfaces of the two lamp beads 262a.

Specific structures of the lamp bead in the light emitter 262 and the light receiver 263 are not limited in this embodiment of this application. For example, the lamp bead in the light emitter 262 may be, for example, a light emitting diode (light emitting diode, LED). The light receiver 263 may be, for example, a photo-diode (photo-diode, PD).

Light emitted by the lamp bead in the light emitter 262 may be visible light, such as green light. In some other embodiments, the light emitted by the lamp bead in the light emitter 262 may be red light, blue light, or the like. This is not specifically limited herein.

There may be one or a plurality of light receivers 263. "Plurality of" means two or more. On this basis, both a quantity of the second light transmissive parts 261b and a quantity of the second light holes 24b may be equal to a quantity of the light receivers 263.

In the embodiment shown in FIG. 2-FIG. 5, a quantity of light receivers 263 is two, and the two light receivers 263 are respectively located on opposite sides of the light emitter 262.

On the basis of the above, refer to FIG. 6, FIG. 6 is a top view of a cover plate 261 in an electronic device 100 shown in FIG. 4. A shape of the first light transmissive part 261a is approximately rectangular, and the first light transmissive part 261a is opposite to the light emitting surface of the light emitter 262. There are two second light transmissive parts 261b, shapes of the two second light transmissive parts 261b are also approximately rectangular, and the two second light transmissive parts 261b are respectively opposite to light incident surfaces of the two light receivers 263. In other embodiments, the first light transmissive part 261a and the second light transmissive part 261b may be in circular, polygonal, or other irregular shapes.

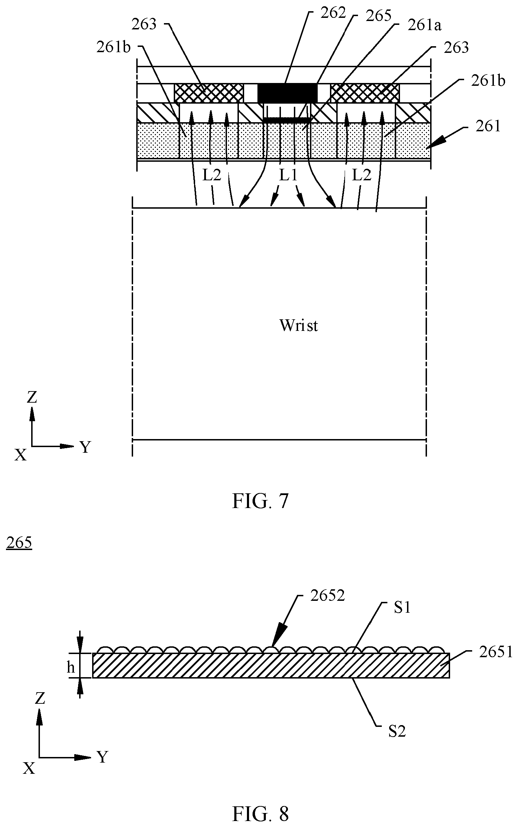

Refer to FIG. 7. FIG. 7 is a schematic diagram of a structure of an electronic device 100 shown in FIG. 4 in a case of being worn on a wrist of a user. When the photoplethysmograph 26 operates, light L1 emitted from the light emitting surface of the light emitter 262 enters the wrist through the first light transmissive part 261a, part of the light is reflected after being absorbed by human blood and tissues at the wrist, to form reflected light L2. The reflected light L2 enters the light incident surface of the light receiver 263 through the second light transmissive part 261b, to receive optical signals.

When the light emitted by the light emitter 262 reaches the cover plate 261, the light may be reflected on surfaces of the cover plate 261 that respectively face the light emitter 262 and face away from the light emitter 262 due to different refractive indexes of the cover plate 261 and air. The greater an incident angle is, the stronger the reflected light is. Based on this, the light emitted by the light emitter 262 is a divergent light beam, not a parallel light beam, and because the surfaces of the cover plate 261 that respectively face the light emitter 262 and face away from the light emitter 262 are approximately planar, the cover plate 261 has a high reflectivity for the light emitted by the light emitter 262. This leads to low light emitting efficiency and less light entering the human body. Moreover, light emitted from the cover plate 261 has a large light emitting angle. This leads to a low maximum intensity of light entering the human body. Therefore, detection accuracy and a perfusion index are affected.

To solve the foregoing problems, refer back to FIG. 2-FIG. 4. The photoplethysmograph 26 further includes an optical lens 265, and the optical lens 265 is disposed on a surface that is of the first light transmissive part 261a and that faces the light emitter 262.

Specifically, the optical lens 265 may be disposed in a part of a region of the surface that is of the first light transmissive part 261a and that faces the light emitter 262, or an entire region of the surface that is of the first light transmissive part 261a and that faces the light emitter 262. Focus on FIG. 6. In this embodiment, an example in which the optical lens 265 is disposed on the entire region of the surface that is of the first light transmissive part 261a and that faces the light emitter 262 is used for description, which cannot be considered as a special limitation to this application.

Material of the optical lens 265 may include at least one of glass, sapphire, polyamide (polyamide), polycarbonate (polycarbonate), polymeric methyl methacrylate (polymeric methyl methacrylate, PMMA), thermoplastic polyurethanes (thermoplastic polyurethanes, TPU), and transparent nylon.

The optical lens 265 may be fixed to the surface that is of the first light transmissive part 261a and that faces the light emitter 262 by gluing, or may be integrally formed with the first light transmissive part 261a, that is, the optical lens 265 and the first light transmissive part 261a are integrated as a structural piece.

Specifically, when the material of the optical lens 265 is polyamide, polycarbonate, polymeric methyl methacrylate, thermoplastic polyurethanes, and transparent nylon, the optical lens 265 may be molded on the first light transmissive part 261a through an integral injection molding process (such as a two-color injection molding process). Specifically, the injection molding process includes stages such as compound die, filling, pressure maintaining, cooling, mold opening, and demolding.

In this way, there is no other medium between the optical lens 265 and the first light transmissive part 261a, which can ensure light emitting efficiency, and isolate mixing light, to avoid light from leaking between the optical lens 265 and the first light transmissive part 261a. In addition, there is no need for an attaching operation, thereby reducing process difficulty.

The optical lens 265 is used to adjust incident angles of light emitted by the light emitter 262 on the surfaces of the cover plate 261 that respectively face the light emitter 262 and face away from the light emitter 262, to reduce reflectivity and increase light energy entering the human body. In addition, the optical lens 265 is further used to reduce a light emitting angle of light emitted by the light emitter 262 in a case of emitting from the cover plate 261, and increase the maximum intensity of light entering the human body. Therefore, measurement accuracy and a perfusion index are improved. In addition, because the optical lens 265 is disposed on the surface that is of the first light transmissive part 261a and that faces the light emitter 262, the optical lens 265 is invisible to a user of the electronic device. In this way, aesthetics of the electronic device can be ensured.

The following focuses on a structure of the optical lens 265.

Refer to FIG. 8. FIG. 8 is an enlarged diagram of an optical lens 265 in an electronic device 100 shown in FIG. 1-FIG. 4. The optical lens 265 includes a light-transmitting substrate 2651 and a microlens array 2652.

The light-transmitting substrate 2651 has a first surface S1 and a second surface S2 opposite to each other. Both the first surface S1 and the second surface S2 are planar, and the first surface S1 is approximately parallel to the second surface S2. When the optical lens 265 is used in the photoplethysmograph 26, the first surface S1 faces the light emitter 262, and the second surface S2 faces the first light transmissive part 261a.

A thickness h of the light-transmitting substrate 2651 is a spacing from the first surface S1 to the second surface S2. In some embodiments, the thickness h of the light-transmitting substrate 2651 may be greater than or equal to 100 nanometers (nm) and less than or equal to 400 nm. Specifically, the thickness h of the light-transmitting substrate 2651 may be 100 nm, 150 nm, 200 nm, 250 nm, 300 nm, 350 nm, or 400 nm. In this way, the thickness h of the light-transmitting substrate 2651 is moderate, which can support the microlens array 2652, and absorb less light, thereby ensuring light emitting efficiency.

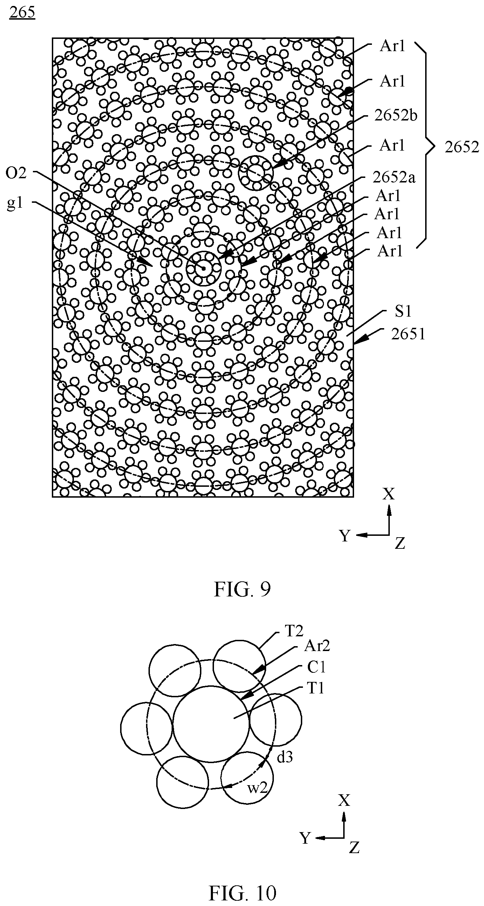

The microlens array 2652 is arranged on the first surface S1. Refer to FIG. 9. FIG. 9 is a schematic diagram of a structure of an optical lens 265 shown in FIG. 8 on a first surface S1. The microlens array 2652 may cover an entire region of the first surface S1, or may be arranged in a part of region of the first surface S1. This application uses an example in which the microlens array 2652 covers the entire region of the first surface S1 for description. The microlens array 2652 includes a plurality of microlenses arranged in an array, and each microlens is a convex sphere protruding from the first surface S1 in a direction away from the second surface S2.

The convex sphere can converge light beams and can reduce incident angles of some light on surfaces of the cover plate 261 that respectively face the light emitter 262 and face away from the light emitter 262. In this way, an amount of light reflected by the cover plate 261 can be reduced. Moreover, the convex sphere can converge light beams and can further reduce a light emitting angle of light emitted from the cover plate 261. However, because of being in an optical path, the microlens may inevitably absorb some light, and energy loss may be caused easily. If an array mode of the microlens is not designed properly, light emitting efficiency may be reduced, light energy and a maximum intensity of light entering the human body may be reduced, and measurement accuracy and a perfusion index may be reduced.

Therefore, the following focuses on an array form of the microlens.

Still refer to FIG. 9. The microlens array 2652 includes a first microlens assembly 2652a and a plurality of second microlens assembly arrays Ar1. In the embodiment shown in FIG. 9, a quantity of second microlens assembly arrays Ar1 is seven. In other embodiments, the quantity of the second microlens assembly arrays Ar1 may be three, four, five, six, or the like. This is not specifically limited herein.

Each of the second microlens assembly arrays Ar1 includes a plurality of second microlens assemblies 2652b arranged around the first microlens assembly 2652a in an array. The plurality of second microlens assembly arrays Ar1 are sequentially arranged along a radial direction of an annular array track of the plurality of second microlens assemblies 2652b.

Structures of the first microlens assembly 2652a and the second microlens assembly 2652b may be the same or different. The following embodiments are described in a case that the first microlens assembly 2652a and the second microlens assembly 2652b are in a same structure, which cannot be considered as a special limitation to this application.

On the basis of the above, refer to FIG. 10. FIG. 10 is a schematic diagram of a structure of a first microlens assembly 2652a or a second microlens assembly 2652b in a microlens array in an optical lens 265 shown in FIG. 9. The first microlens assembly 2652a and the second microlens assembly 2652b each include a first microlens unit C1 and a second microlens array Ar2.

The first microlens unit C1 includes at least one first microlens T1. "At least one" means one or more. In the embodiment shown in FIG. 10, there is one first microlens T1 in the first microlens unit C1. In other embodiments, there may be a plurality of first microlenses T1 in the first microlens unit C1, and "plurality of" means two or more.

The second microlens array Ar2 includes a plurality of second microlenses T2 arranged around the first microlens unit C1 in an array. In the embodiment shown in FIG. 10, a quantity of second microlenses T2 in the second microlens array Ar2 is six. In other embodiments, the quantity of second microlenses T2 may be four, five, eight, ten, twelve, or the like. This is not specifically limited herein.

In the foregoing embodiment, both the first microlens T1 and the second microlens T2 are convex spheres protruding from the first surface S1 in a direction away from the second surface S2.

Due to an array mode of the microlens array, light beams emitted by the light emitter 262 may be converged by using the first microlens T1 and the second microlens T2, to reduce incident angles of some light on surfaces of the cover plate 261 that respectively face the light emitter 262 and face away from the light emitter 262. In this way, an amount of light reflected by the cover plate 261 can be reduced. Moreover, light beams emitted by the light emitter 262 are converged by using the first microlens T1 and the second microlens T2, and a light emitting angle of light emitted from the cover plate 261 can be reduced. In addition, some gaps are formed between two adjacent second microlens assembly arrays Ar1, such as gap g1 in FIG. 9. These gaps are not provided with convex spheres, which can reduce an energy loss of light when passing through a microlens array. Therefore, it is beneficial to improve light emitting efficiency, increase light energy and a maximum intensity of light entering the human body, and improve measurement accuracy and a perfusion index.

In addition, the microlens array 2652 occupies a small height, which is convenient for installation in an electronic device with limited space.

In the foregoing embodiment, optionally, refer back to FIG. 9, two adjacent second microlens assembly arrays Ar1 are arranged at intervals and closely along the radial direction of the annular array track of the plurality of second microlens assemblies 2652b. To be specific, the two adjacent second microlens assembly arrays Ar1 are arranged at intervals along the radial direction of the annular array track of the plurality of second microlens assemblies 2652b, and the two adjacent second microlens assembly arrays Ar1 are arranged closely along the radial direction of the annular array track of the plurality of second microlens assemblies 2652b.

That the two adjacent second microlens assembly arrays Ar1 are arranged at intervals along the radial direction of the annular array track of the plurality of second microlens assemblies 2652b indicates: a radial direction spacing between the two adjacent second microlens assembly arrays Ar1 along the annular array track of the plurality of second microlens assemblies 2652b is greater than or equal to 0. To be specific, for the two adjacent second microlens assembly arrays Ar1, an inner diameter of the second microlens assembly array Ar1 located at an outer layer is greater than or equal to an outer diameter of the second microlens assembly array Ar1 located at an inner layer.

It should be noted that, the outer diameter of the second microlens assembly array Ar1 refers to: in the second microlens assembly array Ar1, a diameter of a circular outer contour formed, on a plurality of second microlens assemblies 2652b, by connecting points farthest from a center of an annular array track of the plurality of second microlens assemblies 2652b. Correspondingly, the inner diameter of the second microlens assembly array Ar1 refers to: in the second microlens assembly array Ar1, a diameter of a circular inner contour formed, on a plurality of second microlens assemblies 2652b, by connecting points nearest from a center of an annular array track of the plurality of second microlens assemblies 2652b.

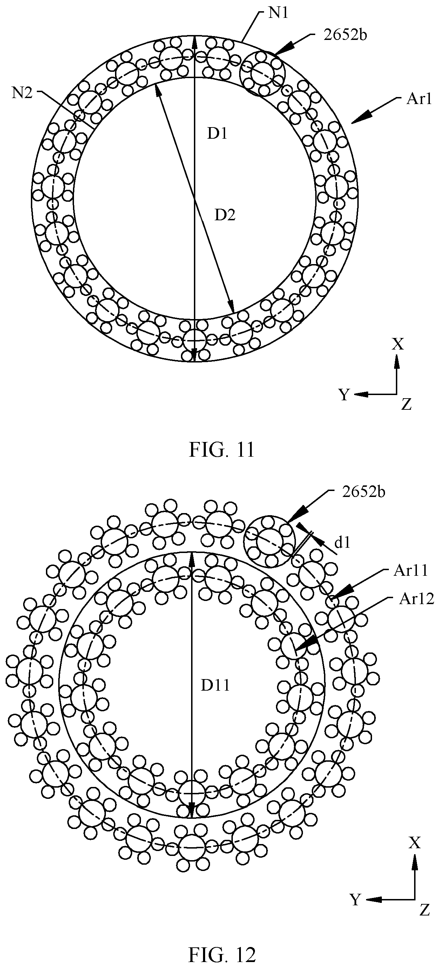

For example, refer to FIG. 11. FIG. 11 is a schematic diagram of a structure of a second microlens assembly array Ar1 in an optical lens 265 shown in FIG. 9. The second microlens assembly array Ar1 refers to: in the second microlens assembly array Ar1, a diameter D1 of a circular outer contour N1 formed, on a plurality of second microlens assemblies 2652b, by connecting points farthest from a center of an annular array track of the plurality of second microlens assemblies 2652b. Correspondingly, an inner diameter of the second microlens assembly array Ar1 refers to: in the second microlens assembly array Ar1, a diameter D2 of a circular inner contour N2 formed, on a plurality of second microlens assemblies 2652b, by connecting points nearest from a center of an annular array track of the plurality of second microlens assemblies 2652b. The inner diameter and the outer diameter of the second microlens assembly array Ar1 described in the following embodiments should be understood in the same manner, and details may not be described again.

In addition, that the two adjacent second microlens assembly arrays Ar1 are arranged closely along the radial direction of the annular array track of the plurality of second microlens assemblies 2652b indicates: for two adjacent second microlens assembly arrays Ar1, after a second microlens assembly array Ar1 located at an outer layer is reduced by one second microlens assembly 2652b, an inner diameter of a new array formed by arranging a remaining quantity of second microlens assemblies 2652b along an annular array is smaller than an outer diameter of a second microlens assembly array Ar1 located at an inner layer while keeping a spacing between the two adjacent second microlens assemblies 2652b unchanged.

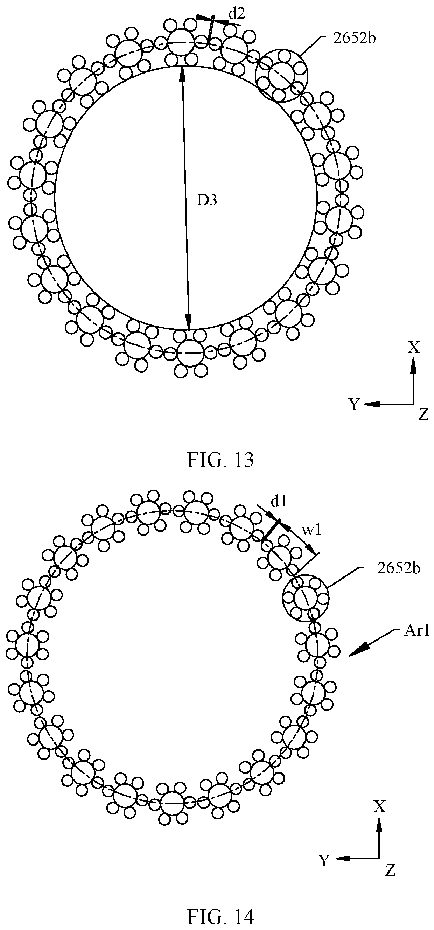

For example, refer to FIG. 12. FIG. 12 is a schematic diagram of a structure of two adjacent second microlens assembly arrays Ar1 in an optical lens 265 shown in FIG. 9. Specifically, the second microlens assembly array located at the outer layer is marked as Ar11, and the second microlens assembly array located at the inner layer is marked as Ar12. A quantity of second microlens assemblies 2652b in the second microlens assembly arrays Ar11 located at the outer layer is nineteen, and an outer diameter of the second microlens assembly arrays Ar12 located at the inner layer is D11. The second microlens assembly arrays Ar11 located at the outer layer has eighteen second microlens assemblies 2652b after reducing one second microlens assembly 2652b. For a new array formed by arranging these eighteen second microlens assemblies 2652b along an annular array, refer to FIG. 13. A spacing d2 between two adjacent second microlens assemblies 2652b in the new array is equal to a spacing d1 between two adjacent second microlens assemblies 2652b in the second microlens assembly array Ar11 located at the outer layer in FIG. 12. "Equal" should be understood as approximately equal. When a difference thereof is less than or equal to 0.01 mm, it may be considered as being equal. An inner diameter of the new array shown in FIG. 13 is D3. D3 is smaller than the outer diameter D11 of the second microlens assembly array Ar12 located at the inner layer. To be specific, a spacing between the second microlens assembly array Ar11 located at the outer layer and the second microlens assembly array Ar12 located at the inner layer is as close as possible.

In addition, refer back to FIG. 9, the first microlens assembly 2652a and an adjacent second microlens assembly array Ar1 are also arranged at intervals and closely along the radial direction of the annular array track of the plurality of second microlens assemblies 2652b. To be specific, the first microlens assembly 2652a and the adjacent second microlens assembly array Ar1 are arranged at intervals along the radial direction of the annular array track of the plurality of second microlens assemblies 2652b, and the first microlens assembly 2652a and the adjacent second microlens assembly array Ar1 are arranged closely along the radial direction of the annular array track of the plurality of second microlens assemblies 2652b.

The "adjacent second microlens assembly array Ar1 "refers to a second microlens assembly array Ar1 which is closest to the first microlens assembly 2652a.

In addition, that the first microlens assembly 2652a and the adjacent second microlens assembly array Ar1 are arranged at intervals along the radial direction of the annular array track of the plurality of second microlens assemblies 2652b indicates: a radial direction spacing between the first microlens assembly 2652a and the adjacent second microlens assembly array Ar1 along the annular array track of the plurality of second microlens assemblies 2652b is greater than or equal to 0. To be specific, an inner diameter of the adjacent second microlens assembly array Ar1 is greater than or equal to an outer diameter of the first microlens assembly 2652a.

In addition, that the first microlens assembly 2652a and the adjacent second microlens assembly array Ar1 are arranged closely along the radial direction of the annular array track of the plurality of second microlens assemblies 2652b indicates: after the adjacent second microlens assembly array Ar1 is reduced by one second microlens assembly 2652b, an inner diameter of a new array formed by arranging a remaining quantity of second microlens assemblies 2652b along an annular array is smaller than the outer diameter of the first microlens assembly 2652a while keeping a spacing between the two adjacent second microlens assemblies 2652b unchanged.

In this way, a quantity of second microlens assembly arrays Ar1 arranged on the first surface S1 is large, light beams emitted by the light emitter 262 can be converged as much as possible, and incident angles of some light on surfaces of the cover plate 261 that respectively face the light emitter 262 and face away from the light emitter 262 are reduced. Therefore, an amount of light reflected by the cover plate 261 can be reduced. Moreover, light beams emitted by the light emitter 262 can be converged as much as possible, and a light emitting angle of light emitted from the cover plate 261 can be reduced. Therefore, light emitting efficiency can be improved, light energy and a maximum intensity of light entering the human body are increased, and measurement accuracy and a perfusion index are improved.

In addition, in some embodiments, refer back to FIG. 9, in each second microlens assembly array Ar1, a plurality of second microlens assemblies 2652b are arranged at intervals and closely along an annular array track of the plurality of second microlens assemblies 2652b. To be specific, in each second microlens assembly array Ar1, a plurality of second microlens assemblies 2652b are arranged at intervals along an annular array track of the plurality of second microlens assemblies 2652b, and the plurality of second microlens assemblies 2652b are arranged closely along the annular array track of the plurality of second microlens assemblies 2652b.

That a plurality of second microlens assemblies 2652b are arranged at intervals along an annular array track of the plurality of second microlens assemblies 2652b indicates: in the plurality of second microlens assemblies 2652b, a spacing between two adjacent second microlens assemblies 2652b along the annular array track of the plurality of second microlens assemblies 2652b is greater than or equal to 0. In this way, there is no overlap between two adjacent second microlens assemblies 2652b. That the plurality of second microlens assemblies 2652b are arranged closely along the annular array track of the plurality of second microlens assemblies 2652b indicates: in the plurality of second microlens assemblies 2652b, a sum of spacings between two adjacent second microlens assemblies 2652b along the annular array track of the plurality of second microlens assemblies 2652b is smaller than a width of a single second microlens assembly 2652b along the annular array track.

For example, refer to FIG. 14. FIG. 14 is a schematic diagram of a structure of a second microlens assembly array Ar1 in an optical lens 265 shown in FIG. 9. A quantity of second microlens assemblies 2652b in the second microlens assembly array Ar1 is nineteen. It is assumed that a spacing between every two adjacent second microlens assemblies 2652b along the annular array track of the second microlens assembly array Ar1 is d1, where d1≥0 mm. Nineteen spacings d1 are formed for the nineteen second microlens assemblies 2652b, and a sum of the nineteen spacings d1 is approximately 19×d1. On this basis, still refer to FIG. 14. It is assumed that a width of a single second microlens assembly 2652b along the annular array track of the second microlens assembly array Ar1 is w1, and then 19×d1<w1. In other words, when a diameter of the annular array track of the second microlens assembly array Ar1 is constant, a quantity of second microlens assemblies 2652b in the second microlens assembly array Ar1 has reached saturation, and no second microlens assembly 2652b can be added.

In this way, a quantity of second microlens assemblies 2652b arranged in each second microlens assembly array Ar1 is large, light beams emitted by the light emitter 262 can be converged as much as possible, and incident angles of some light on surfaces of the cover plate 261 that respectively face the light emitter 262 and face away from the light emitter 262 are reduced. Therefore, an amount of light reflected by the cover plate 261 can be reduced. Moreover, light beams emitted by the light emitter 262 can be converged as much as possible, and a light emitting angle of light emitted from the cover plate 261 can be reduced. Therefore, light emitting efficiency can be improved, light energy and a maximum intensity of light entering the human body are increased, and measurement accuracy and a perfusion index are improved.

In addition, in some embodiments, refer back to FIG. 10, in the first microlens assembly 2652a and the second microlens assembly 2652b, the second microlens array Ar2 and the first microlens unit C1 are arranged at intervals and closely along a radial direction of an annular array track of the plurality of second microlenses T2. To be specific, the second microlens array Ar2 and the first microlens unit C1 are arranged at intervals along the radial direction of the annular array track of the plurality of second microlenses T2, and the second microlens array Ar2 and the first microlens unit C1 are arranged closely along the radial direction of the annular array track of the plurality of second microlenses T2.

That the second microlens array Ar2 and the first microlens unit C1 are arranged at intervals along the radial direction of the annular array track of the plurality of second microlenses T2 indicates: a radial direction spacing between the second microlens array Ar2 and the first microlens unit C1 along the annular array track of the plurality of second microlenses T2 is greater than or equal to 0. In other words, an inner diameter of the second microlens array Ar2 is greater than or equal to a maximum width of the first microlens unit C1.

It should be noted that, the inner diameter of the second microlens array Ar2 refers to: in the second microlens array Ar2, a diameter of a circular inner contour formed, on a plurality of second microlenses T2, by connecting points nearest from a center of an annular array track of the plurality of second microlenses T2.

On the basis of the above, it should be noted that, the maximum width of the first microlens unit C1 refers to a maximum width of an occupied region of the first microlens unit C1 on the first surface S1. When there is one first microlens T1 in the first microlens unit C1, the maximum width is a diameter of the occupied region of the first microlens T1 on the first surface S1. When there are a plurality of first microlenses T1 that are in the first microlens unit C1 and that are arranged in a row, the maximum width is a sum of a diameter of an occupied region of the plurality of first microlenses T1 on the first surface S1 and a spacing between two adjacent first microlenses T1.

In addition, that the second microlens array Ar2 and the first microlens unit C1 are arranged closely along the radial direction of the annular array track of the plurality of second microlenses T2 indicates: after the second microlens array Ar2 is reduced by one second microlens T2, an inner diameter of a new array formed by arranging a remaining quantity of second microlenses T2 along an annular array is smaller than a maximum occupied width of the first microlens unit C1 while keeping a spacing between two adjacent second microlenses T2 unchanged. To be specific, a spacing between the second microlens array Ar2 and the first microlens unit C1 is as close as possible.

In this way, an occupied area of the first microlens assembly 2652a and the second microlens assembly 2652b on the first surface S1 is small, and a quantity of first microlens assemblies 2652a and second microlens assemblies 2652b that can be arranged on the first surface S1 is large. Light beams emitted by the light emitter 262 can be converged as much as possible, and incident angles of some light on surfaces of the cover plate 261 that respectively face the light emitter 262 and face away from the light emitter 262 are reduced. Therefore, an amount of light reflected by the cover plate 261 can be reduced. Moreover, light beams emitted by the light emitter 262 can be converged as much as possible, and a light emitting angle of light emitted from the cover plate 261 can be reduced. Therefore, light emitting efficiency can be improved, light energy and a maximum intensity of light entering the human body are increased, and measurement accuracy and a perfusion index are improved.

In addition, in some embodiments, still refer back to FIG. 10, in the second microlens array Ar2, a plurality of second microlenses T2 are also arranged at intervals and closely along an annular array track of the plurality of second microlenses T2. To be specific, in the second microlens array Ar2, the plurality of second microlenses T2 are arranged at intervals along the annular array track of the plurality of second microlenses T2, and the plurality of second microlenses T2 are arranged closely along the annular array track of the plurality of second microlenses T2.

That the plurality of second microlenses T2 are arranged at intervals along the annular array track of the plurality of second microlenses T2 indicates: in the plurality of second microlenses T2, a spacing between two adjacent second microlenses T2 along the annular array track of the plurality of second microlenses T2 is greater than or equal to 0. In this way, there is no overlap between two adjacent second microlenses T2.

In addition, that the plurality of second microlenses T2 are arranged closely along the annular array track of the plurality of second microlenses T2 indicates: in the plurality of second microlenses T2, a sum of spacings d3 between two adjacent second microlenses T2 along the annular array track of the plurality of second microlenses T2 is smaller than a width w2 (namely, a diameter of an occupied region of the second microlens T2 on the first surface S1) of a single second microlens T2 along the annular array track. In other words, when a diameter of the annular array track of the second microlens array Ar2 is constant, a quantity of second microlenses T2 in the second microlens array Ar2 has reached saturation, and no second microlens T2 can be added.

In this way, the quantity of second microlenses T2 arranged in the second microlens array Ar2 is large, light beams emitted by the light emitter 262 can be converged as much as possible, and incident angles of some light on surfaces of the cover plate 261 that respectively face the light emitter 262 and face away from the light emitter 262 are reduced. Therefore, an amount of light reflected by the cover plate 261 can be reduced. Moreover, light beams emitted by the light emitter 262 can be converged as much as possible, and a light emitting angle of light emitted from the cover plate 261 can be reduced. Therefore, light emitting efficiency can be improved, light energy and a maximum intensity of light entering the human body are increased, and measurement accuracy and a perfusion index are improved.

The foregoing uses FIG. 10 as an example to describe structures of the first microlens assembly 2652a and the second microlens assembly 2652b in the optical lens 265 provided in the embodiments of this application, and the following describes two other embodiments of the first microlens assembly 2652a and the second microlens assembly 2652b.

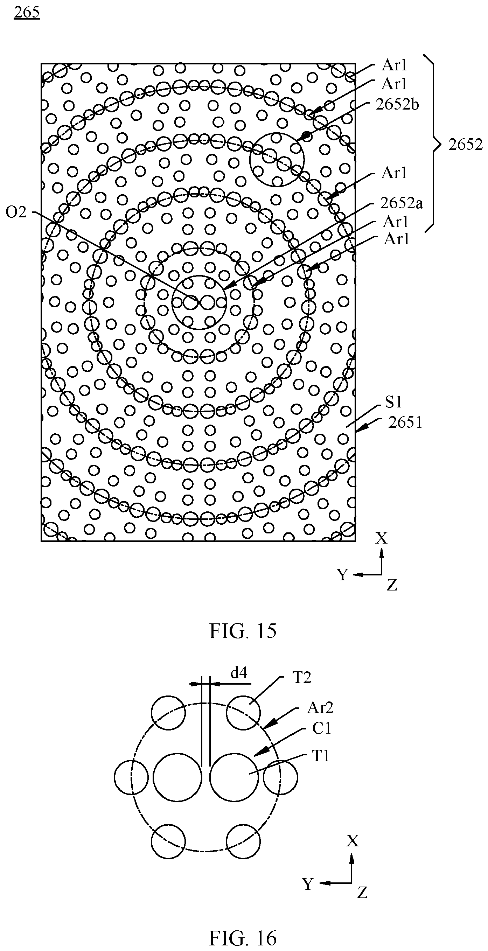

Specifically, refer to FIG. 15 and FIG. 16. FIG. 15 is a schematic diagram of a structure of an optical lens 265 on a first surface S1 according to some other embodiments of this application, and FIG. 16 is a schematic diagram of a structure of a first microlens assembly 2652a or a second microlens assembly 2652b in a microlens array in an optical lens 265 shown in FIG. 15. A difference between the first microlens assembly 2652a and the second microlens assembly 2652b shown in this embodiment and the first microlens assembly 2652a and the second microlens assembly 2652b shown in FIG. 8 includes: in this embodiment, there is a plurality of first microlenses T1 in the first microlens unit C1. Specifically, in the embodiment shown in FIG. 16, there are two first microlenses T1 in the first microlens unit C1. In other embodiments, a quantity of the first microlenses T1 in the first microlens unit C1 may be three, four, five, or the like. This is not specifically limited herein. The plurality of first microlenses T1 are arranged in a row. Moreover, focus on FIG. 15, an arrangement direction of a plurality of first microlenses T1 in the second microlens assembly 2652b is consistent with an extension direction of an annular array track of a second microlens assembly array Ar1 in which the second microlens assembly 2652b is located. "Consistent" should be understood as approximately consistent, and two directions can be regarded as consistent if an included angle between the two directions is less than or equal to 5°. In this way, it is convenient for a plurality of first microlenses T1 to be positioned in the first surface S1, which is conducive to reducing design difficulty.

In the foregoing embodiment, optionally, the plurality of first microlenses T1 in the first microlens unit C1 are arranged at intervals and closely. To be specific, in the first microlens unit C1, the plurality of first microlenses T1 are arranged at intervals, and the plurality of first microlenses T1 are arranged closely.

That the plurality of first microlenses T1 are arranged at intervals indicates: in the plurality of first microlenses T1, a spacing d4 between two adjacent first microlenses T1 is greater than or equal to 0. In addition, that the plurality of first microlenses T1 are arranged closely indicates: a spacing d4 between two adjacent first microlenses T1 is less than or equal to 0.003 millimeters (mm). Specifically, the spacing d4 may be 0.001 mm, 0.002 mm, or 0.003 mm.

In this way, an occupied area of the first microlens unit C1 on the first surface S1 is small, which is conducive to reducing the occupied area of the first microlens assembly 2652a and the second microlens assembly 2652b on the first surface S1, so that a quantity of first microlens assemblies 2652a and second microlens assemblies 2652b that can be arranged on the first surface S1 is large. Light beams emitted by the light emitter 262 can be converged as much as possible, and incident angles of some light on surfaces of the cover plate 261 that respectively face the light emitter 262 and face away from the light emitter 262 are reduced. Therefore, an amount of light reflected by the cover plate 261 can be reduced. Moreover, light beams emitted by the light emitter 262 can be converged as much as possible, and a light emitting angle of light emitted from the cover plate 261 can be reduced. Therefore, light emitting efficiency can be improved, light energy and a maximum intensity of light entering the human body are increased, and measurement accuracy and a perfusion index are improved.

A similarity between the first microlens assembly 2652a and the second microlens assembly 2652b shown in FIG. 15 and FIG. 16 and the first microlens assembly 2652a and the second microlens assembly 2652b shown in FIG. 9 and FIG. 10 includes: the second microlens array Ar2 and the first microlens unit C1 are arranged at intervals and closely along the radial direction of the annular array track of the plurality of second microlenses T2.

In this way, an occupied area of the first microlens assembly 2652a and the second microlens assembly 2652b on the first surface S1 is small, and a quantity of first microlens assemblies 2652a and second microlens assemblies 2652b that can be arranged on the first surface S1 is large. Light beams emitted by the light emitter 262 can be converged as much as possible, and incident angles of some light on surfaces of the cover plate 261 that respectively face the light emitter 262 and face away from the light emitter 262 are reduced. Therefore, an amount of light reflected by the cover plate 261 can be reduced. Moreover, light beams emitted by the light emitter 262 can be converged as much as possible, and a light emitting angle of light emitted from the cover plate 261 can be reduced. Therefore, light emitting efficiency can be improved, light energy and a maximum intensity of light entering the human body are increased, and measurement accuracy and a perfusion index are improved.

A difference between the first microlens assembly 2652a and the second microlens assembly 2652b shown in FIG. 15 and FIG. 16 and the first microlens assembly 2652a and the second microlens assembly 2652b shown in FIG. 9 and FIG. 10 further includes: in the optical lens 265 shown in FIG. 15 and FIG. 16, in the second microlens array Ar2, a plurality of second microlenses T2 are arranged not closely along the annular array track of the plurality of second microlenses T2. Optionally, focus on FIG. 16, a quantity of second microlenses T2 in the second microlens array Ar2 is six. A quantity of second microlenses T2 arranged in the second microlens array Ar2 in this structure is small, which can reduce structural complexity and costs of the optical lens 265.

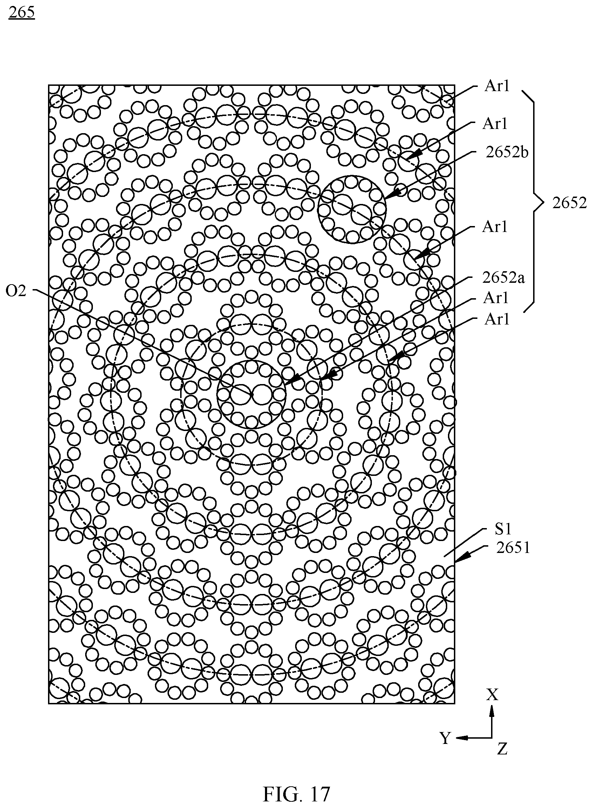

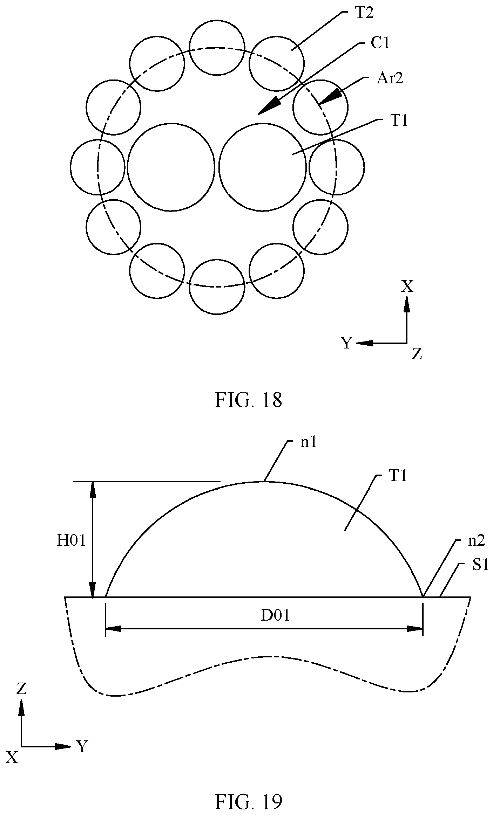

Refer to FIG. 17 and FIG. 18. FIG. 17 is a schematic diagram of a structure of an optical lens 265 on a first surface S1 according to some other embodiments of this application, and FIG. 18 is a schematic diagram of a structure of a first microlens assembly 2652a or a second microlens assembly 2652b in a microlens array in an optical lens 265 shown in FIG. 17. A difference between the first microlens assembly 2652a and the second microlens assembly 2652b shown in this embodiment and the first microlens assembly 2652a and the second microlens assembly 2652b shown in FIG. 15 and FIG. 16 is: in this embodiment, in the second microlens array Ar2, a plurality of second microlenses T2 are arranged closely along the annular array track of the plurality of second microlenses T2. Specifically, a quantity of second microlenses T2 in the second microlens array Ar2 is twelve.

In this way, the quantity of second microlenses T2 arranged in the second microlens array Ar2 is large, light beams emitted by the light emitter 262 can be converged as much as possible, and incident angles of some light on surfaces of the cover plate 261 that respectively face the light emitter 262 and face away from the light emitter 262 are reduced. Therefore, an amount of light reflected by the cover plate 261 can be reduced. Moreover, light beams emitted by the light emitter 262 can be converged as much as possible, and a light emitting angle of light emitted from the cover plate 261 can be reduced. Therefore, light emitting efficiency can be improved, light energy and a maximum intensity of light entering the human body are increased, and measurement accuracy and a perfusion index are improved.

In other embodiments, a plurality of first microlenses T1 in the first microlens unit C1 may also be arranged in a form of triangle, quadrangle, or the like, which is not specifically limited herein.

In any one of the foregoing embodiments, a size of the first microlens T1 and a size of the second microlens T2 may be the same or different, which is not specifically limited herein.

In the embodiment shown in FIG. 10, FIG. 16, or FIG. 18, the size of the first microlens T1 is larger than the size of the second microlens T2.

Optionally, refer to FIG. 19. FIG. 19 is a side view of a structure including a single first microlens T1 and a light-transmitting substrate 2651 in a first microlens assembly 2652a or a second microlens assembly 2652b shown in FIG. 10, FIG. 16, or FIG. 18. A diameter D01 of an occupied region of the first microlens T1 on the first surface S1 is greater than or equal to 0.1 mm and less than or equal to 0.15 mm. Specifically, the diameter D01 may be 0.1 mm, 0.11 mm, 0.12 mm, 0.13 mm, 0.14 mm, or 0.15 mm. A curvature of an end n1 that is of the first microlens T1 and that is away from the first surface S1 is 0 mm⁻¹. A curvature of an end n2 that is of the first microlens T1 and that is connected to the first surface S1 is greater than or equal to 8 mm⁻¹ and less than or equal to 10 mm⁻¹. Specifically, the curvature of the end n2 that is of the first microlens T1 and that is connected to the first surface S1 may be 8 mm⁻¹, 9 mm⁻¹, or 10 mm⁻¹. A height H01 of the first microlens T1 protruding from the first surface S1 is greater than or equal to 0.0252 mm and less than or equal to 0.034 mm. Specifically, the height H01 may be 0.0252 mm, 0.026 mm, 0.027 mm, 0.028 mm, 0.029 mm, 0.030 mm, 0.031 mm, 0.032 mm, 0.033 mm, or 0.034 mm.

In this way, the first microlens T1 can converge light beams better and can reduce incident angles of some light on surfaces of the cover plate 261 that respectively face the light emitter 262 and face away from the light emitter 262. Therefore, an amount of light reflected by the cover plate 261 can be reduced. Moreover, the first microlens T1 can converge light beams better, and can further reduce a light emitting angle of light emitted from the cover plate 261. Therefore, light energy and a maximum intensity of light entering the human body are increased, and measurement accuracy and a perfusion index are improved.

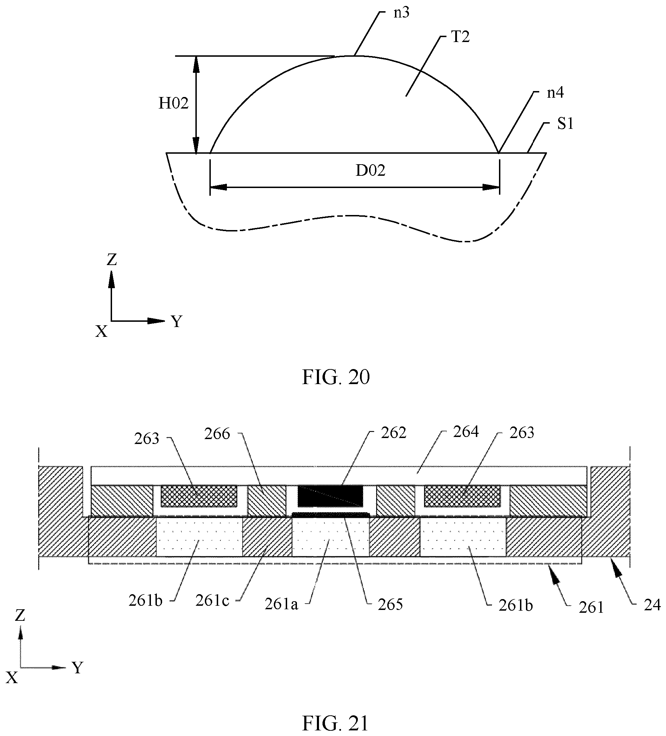

Further, optionally, refer to FIG. 20. FIG. 20 is a side view of a structure including a single second microlens T2 and a light-transmitting substrate 2651 in a first microlens assembly 2652a or a second microlens assembly 2652b shown in FIG. 10, FIG. 16, or FIG. 18. A diameter D02 of an occupied region of the second microlens T2 on the first surface S1 is greater than or equal to 0.05 mm and less than or equal to 0.1 mm. Specifically, the diameter D02 may be 0.05 mm, 0.06 mm, 0.07 mm, 0.08 mm, 0.09 mm, or 0.1 mm. A curvature of an end n3 that is of the second microlens T2 and that is away from the first surface S1 is 0 m⁻¹. A curvature of an end that is of the second microlens T2 and that is connected to the first surface S1 may be greater than or equal to 5 mm⁻¹ and less than or equal to 40 mm⁻¹. Specifically, a curvature of an end n4 that is of the second microlens T2 and that is connected to the first surface S1 may be 5 mm⁻¹, 10 mm⁻¹, 15 mm⁻¹, 20 mm⁻¹, 25 mm⁻¹, 30 mm⁻¹, 35 mm⁻¹, or 40 mm⁻¹. A height H02 of the second microlens T2 protruding from the first surface S1 is greater than or equal to 0.01 mm and less than or equal to 0.0175 mm. Specifically, the height H02 may be 0.01 mm, 0.011 mm, 0.012 mm, 0.013 mm, 0.014 mm, 0.015 mm, 0.016 mm, 0.017 mm, or 0.0175 mm.

In this way, the second microlens T2 can converge light beams better and can reduce incident angles of some light on surfaces of the cover plate 261 that respectively face the light emitter 262 and face away from the light emitter 262. Therefore, an amount of light reflected by the cover plate 261 can be reduced. Moreover, the second microlens T2 can converge light beams better, and can further reduce a light emitting angle of light emitted from the cover plate 261. Therefore, light energy and a maximum intensity of light entering the human body are increased, and measurement accuracy and a perfusion index are improved.

In addition, a microlens array is arranged by combining first microlenses T1 and second microlenses T2 with different sizes, which can converge light beams better, to reduce an amount of light reflected by the cover plate 261, and reduce a light emitting angle of light emitted from the cover plate 261. In addition, some gaps are formed in the microlens array. These gaps are not provided with convex spheres, which can reduce an energy loss of light when passing through a microlens array. Therefore, it is beneficial to improve light emitting efficiency, increase light energy and a maximum intensity of light entering the human body, and improve measurement accuracy and a perfusion index.

The foregoing describes a plurality of structures of the optical lens 265. When the optical lens 265 is disposed on the photoplethysmograph 26, an array center O2 (refer to FIG. 6) of a microlens array in the optical lens 265 is aligned with a center O1 of a light emitting surface of the light emitter 262. "Aligned with" should be understood as approximately aligned with. When a lateral spacing between the array center O2 and the center O1 is less than or equal to 0.1 mm, it can be considered that the array center O2 is aligned with the center O1.

In this way, light emitted by the light emitter 262 can be converged better, an amount of light that is emitted by the light emitter 262 and that is reflected by the cover plate 261 can be reduced, and a light emitting angle of light emitted from the cover plate 261 can be reduced.

To verify performance of the optical lens 265 provided in the foregoing embodiments, the optical lenses 265 shown in FIG. 9, FIG. 15, and FIG. 17 are used in the photoplethysmograph 26 separately, and the center of the microlens array in the optical lens 265 is enabled to be approximately aligned with a center of a light emitting surface of the light emitter 262, to obtain four performance indicators: light emitting efficiency, a light emitting angle, a maximum intensity of light, and a perfusion index (first PI value and second PI value) detected by two light receivers 263, which are recorded in Table 1 below. The light emitting angle refers to an angle of light emitted from the cover plate 261, and the maximum intensity of light refers to a maximum intensity of light entering the human body, where the maximum intensity of light may be measured on an outer surface of the cover plate 261.

Specifically, performance indicators of the optical lens 265 shown in FIG. 9 are recorded in a row of "Embodiment 1" in Table 1, performance indicators of the optical lens 265 shown in FIG. 15 are recorded in a row of "Embodiment 2" in Table 1, and performance indicators of the optical lens 265 shown in FIG. 17 are recorded in a row of "Embodiment 3" in Table 1.

Moreover, a photoplethysmograph 26 without the optical lens 265 is used as a comparison scheme, and obtained light emitting efficiency, a light emitting angle, a maximum intensity of light, and a perfusion index (a first PI value and a second PI value) detected by two light receivers 263 are recorded in a "Comparative example" row of Table 1.

**Table 1**

| | Light emitting efficiency | Light emitting angle | Maximum intensity of light | First PI value | Second PI value |
|---|---|---|---|---|---|
| Comparative example | 79.628% | 100° | 0.00526 W/Sr | 0.021316062 | 0.022574238 |
| Embodiment 1 | 75.233% | 93° | 0.005426 W/Sr | 0.021699939 | 0.022718618 |
| Embodiment 2 | 73.230% | 95° | 0.0052989 W/Sr | 0.021833321 | 0.02259 |
| Embodiment 3 | 70.859% | 95° | 0.0052617 W/Sr | 0.022441 | 0.022725 |

When performance of the photoplethysmograph 26 is evaluated, among the four performance indicators: light emitting efficiency, a light emitting angle, an intensity of emitted light, and a perfusion index, the perfusion index should be given priority, followed by the light emitting angle and the maximum intensity of emitted light, and finally the light emitting efficiency is considered. Therefore, it can be seen from Table 1, compared with "Comparative example", in "Embodiment 1", the perfusion index is improved by 3.8% at the maximum, and the first PI value and the second PI value are improved by 1.203% on average. In "Embodiment 2", the perfusion index is improved by 2.47% at the maximum, and the first PI value and the second PI value are improved by 1.2036% on average. In "Embodiment 3", the perfusion index is improved by 5.28% at the maximum, and the first PI value and the second PI value are improved by 2.91% on average. Therefore, the optical lens 265 provided in the embodiments of this application can improve the performance of the photoplethysmograph 26. Instead of greatly reducing light emitting and receiving efficiency of the photoplethysmograph 26, the optical lens 265 provided in this application may improve the perfusion index of the photoplethysmograph 26, specifically by 1%-6%.

Refer to FIG. 21. FIG. 21 is a schematic diagram of a structure of a photoplethysmograph 26 in an electronic device 100 according to some other embodiments of this application. A difference between the photoplethysmograph 26 shown in this embodiment and the photoplethysmograph 26 shown in FIG. 4 includes: in this embodiment, a cover plate 261 further includes a light shielding part 261c, the light shielding part 261c is opaque to light, and the light shielding part 261c is located between at least a first light transmissive part 261a and a second light transmissive part 261b. In this way, the light shielding part 261c may be used to prevent some light emitted by a light emitter 262 from directly entering a collection range of a light receiver 263 without passing through a human body part to be detected, to avoid affecting detection accuracy of the photoplethysmograph 26.

On the basis of the foregoing embodiments, optionally, still refer to FIG. 21. Other parts of the cover plate 261 other than the first light transmissive part 261a and the second light transmissive part 261b are light shielding parts 261c. In this way, the light shielding part 261c is used to prevent, as much as possible, some light emitted by the light emitter 262 from directly entering a collection range of the light receiver 263 without passing through a human body part to be detected, to avoid affecting detection accuracy of the photoplethysmograph 26.

In some embodiments, the light shielding part 261c may be plastic. Specifically, material of the light shielding part 261c includes, but is not limited to, metal material such as stainless steel and plastics such as polycarbonate (polycarbonate, PC), PC+ glass fiber, and ABS plastic (acrylonitrile butadiene styrene plastic). The light shielding part 261c is integrated with a lower casing 24, and the light shielding part 261c is integrally formed with the first light transmissive part 261a and the second light transmissive part 261b. Specifically, the light shielding part 261c and the lower casing 24 may be formed on the first light transmissive part 261a and the second light transmissive part 261b by using an injection molding process. In this way, the electronic device 100 includes fewer parts, which is convenient to simplify assembly difficulty.

In some embodiments, still refer to FIG. 21, a surface that is of a circuit board 264 and that faces the cover plate 261 is also provided with a retaining wall 266, and the retaining wall 266 is located between at least the light emitter 262 and the light receiver 263. With the retaining wall 266, some light emitted by the light emitter 262 may also be prevented from directly entering the collection range of the light receiver 263 without passing through the human body part to be detected, to avoid affecting detection accuracy of the photoplethysmograph 26.

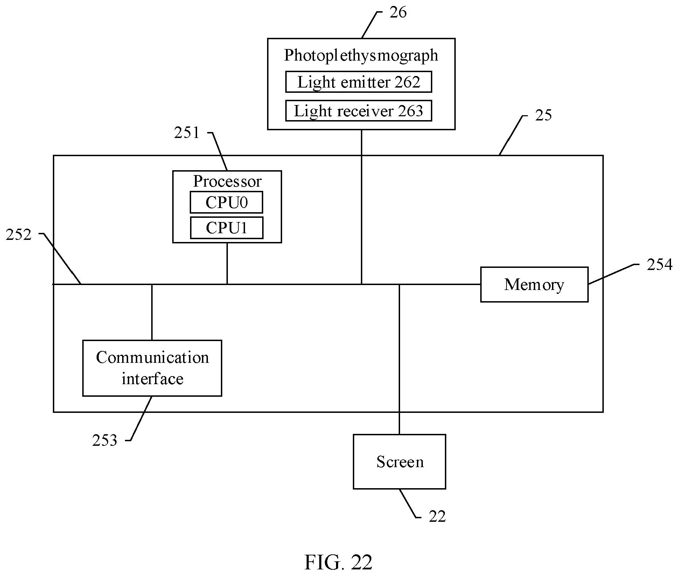

Refer to FIG. 22. FIG. 22 is a structural block diagram of a circuit board assembly 25 in an electronic device 100 according to some embodiments of this application. The circuit board assembly 25 includes at least one processor 251, a communication bus 252, at least one communication interface 253, and a memory 254.

It can be understood that FIG. 20 shows examples of some components included in the circuit board assembly 25. The circuit board assembly 25 may include more or fewer components than those shown in FIG. 20, or some components may be combined, or a different component deployment may be used. Although not shown, the circuit board assembly 25 may further include a Bluetooth module, a global positioning system (Global Positioning System, GPS) module, and the like. Details are not described herein again.

The processor 251 is in a communication connection with at least one communication interface 253, a memory 254, and a screen 22 through a communication bus 252. The processor 251 may be a central processing unit (central processing unit, CPU), or may be another general-purpose processor, a digital signal processor (digital signal processor, DSP), an application specific integrated circuit (application specific integrated circuit, ASIC), a field-programmable gate array (field-programmable gate array, FPGA), or another programmable logic device, a discrete gate or a transistor logic device, a discrete hardware component, or the like. The general-purpose processor may be a microprocessor, or the processor may be any conventional processor or the like. The processor is a control center of the electronic device 100, and is connected to all parts of the entire electronic device 100 by using various interfaces and lines.

The communication bus 252 may include a channel, to transmit information between the foregoing components.

The communication interface 253 uses any apparatus of a transceiver type to communicate with another device or a communication network such as an Ethernet, a radio access network (radio access network, RAN), a wireless local area network (wireless local area networks, WLAN), and the like.

The memory 254 may be used to store computer programs and/or modules, and the processor 251 realizes various functions of the circuit board assembly 25 by running or executing the computer programs and/or modules stored in the memory 254 and calling data stored in the memory 254. The memory 254 may mainly include a program storage area and a data storage area. The program storage area may store an operating system, and an application required by a plurality of functions (for example, a sound playing function or an image playing function). The data storage area may store data (for example, audio data and a phone book) created according to the use of the circuit board assembly 25, and the like. In addition, the memory 254 may include a high-speed random access memory, or may include a non-volatile memory such as a hard disk, an internal storage, a plug-in hard disk, a smart media card (smart media card, SMC), a secure digital (secure digital, SD) card, a flash card (flash card), a plurality of magnetic disk storage devices, a flash storage device, or another volatile solid-state storage device. The memory 254 may exist independently, and is connected to the processor 251 by using a communication bus 252. The memory 254 may alternatively be integrated with the processor 251.

During specific implementation, in an embodiment, the processor 251 may include one or more CPUs, for example, a CPU0 and a CPU1 in the figure.

During specific implementation, in an embodiment, the circuit board assembly 25 may include a plurality of processors 251, for example, a CPU0 and a CPU1 in FIG. 22. Each of these processors may be a single-core (single-CPU) processor, or may be a multi-core (multi-CPU) processor. The processor 251 herein may be one or more devices or circuits, and/or a processing core configured to process data (for example, computer program instructions).

In an embodiment of this application, the processor 251 is electrically connected to a light emitter 262, a light receiver 263, and a screen 22. When a user triggers a photoplethysmograph to start operating, the processor 251 controls the light emitter 262 to emit light, and part of the light is reflected after being absorbed by human blood and tissues of the user. The light receiver 263 may receive reflected light passing through the body of the user, convert an optical signal into an electrical signal, and send the electrical signal to the processor 251. The processor 251 can determine a change of a heart rate of a wearer, and the like based on the reflected light received by the light receiver 263, and display the change on the screen 22.

The foregoing embodiments describe application scenarios of the optical lens 265 on the photoplethysmograph 26. In some other embodiments, the optical lens 265 may also be used in a light emitting side of a fill light of a device such as a camera or a camera device, to converge a fill light source. In addition, the optical lens 265 may also be used in a light emitting side of a light source of a device such as a projector, to converge illuminating beams. This is not specifically limited herein.

In the descriptions of this specification, the specific features, structures, materials, or characteristics may be combined in a proper manner in any one or more of the embodiments or examples.

Finally, it should be noted that: the foregoing embodiments are merely used for describing the technical solutions of this application, but are not intended to limit this application. Although this application is described in detail with reference to the foregoing embodiments, it should be appreciated by a person of ordinary skill in the art that, modifications may still be made to the technical solutions described in the foregoing embodiments, or equivalent replacements may be made to the part of the technical features.

## Claims

1. An optical lens (265), comprising:
a light-transmitting substrate (2651), wherein the light-transmitting substrate (2651) is provided with a first surface (S1) and a second surface (S2) opposite to each other; and
a microlens array (2652), wherein the microlens array (2652) is arranged on the first surface (S1), the microlens array (2652) comprises a first microlens assembly (2652a) and a plurality of second microlens assembly arrays (Ar1), each of the second microlens assembly arrays (Arl) comprises a plurality of second microlens assemblies (2652b) arranged around the first microlens assembly (2652a) in an array, and the plurality of second microlens assembly arrays (Arl) are sequentially arranged along a radial direction of an annular array track of the plurality of second microlens assemblies (2652b), wherein
both the first microlens assembly (2652a) and the second microlens assembly (2652b) comprise a first microlens unit (C1) and a second microlens array (Ar2), the first microlens unit (C1) comprises at least one first microlens (T1), and the second microlens array (Ar2) comprises a plurality of second microlenses (T2) arranged around the first microlens unit (C1) in an array; and
both the first microlens (T1) and the second microlens (T2) are convex spheres protruding from the first surface (S1) in a direction away from the second surface (S2).

2. The optical lens according to claim 1, wherein two adjacent second microlens assembly arrays are arranged at intervals and closely along the radial direction of the annular array track of the plurality of second microlens assemblies, and the first microlens assembly and the adjacent second microlens assembly array are arranged at intervals and closely along the radial direction of the annular array track of the plurality of second microlens assemblies.

3. The optical lens according to claim 1 or 2, wherein a plurality of second microlens assemblies in each second microlens assembly array are also arranged at intervals and closely along an annular array track of the plurality of second microlens assemblies.

4. The optical lens according to any one of claims 1 to 3, wherein the second microlens array and the first microlens unit are arranged at intervals and closely along a radial direction of an annular array track of the plurality of second microlenses.

5. The optical lens according to any one of claims 1 to 4, wherein the plurality of second microlenses in the second microlens array are also arranged at intervals and closely along the annular array track of the plurality of second microlenses.

6. The optical lens according to claim 5, wherein a quantity of the first microlenses in the first microlens unit is one, and a quantity of the second microlenses in the second microlens array is six.

7. The optical lens according to claim 4 or 5, wherein there are a plurality of the first microlenses in the first microlens unit, the plurality of first microlenses are arranged in a row, and an arrangement direction of the plurality of first microlenses in the second microlens assembly is consistent with an extension direction of an annular array track of a second microlens assembly array in which the second microlens assembly is located.

8. The optical lens according to claim 7, wherein in the first microlens assembly and the second microlens assembly, the plurality of first microlenses are arranged at intervals and closely.

9. The optical lens according to claim 8, wherein in both the first microlens assembly and the second microlens assembly, a quantity of the first microlenses is two, and a quantity of the second microlenses in the second microlens array is twelve.

10. The optical lens according to claim 8, wherein in both the first microlens assembly and the second microlens assembly, a quantity of the first microlenses is two, and a quantity of the second microlenses in the second microlens array is six.

11. The optical lens according to any one of claims 1 to 10, wherein a diameter of an occupied region of the first microlens on the first surface is greater than or equal to 0.1 mm and less than or equal to 0.15 mm, a curvature of an end that is of the first microlens and that is away from the first surface is 0 mm⁻¹, a curvature of an end that is of the first microlens and that is connected to the first surface is greater than or equal to 8 mm⁻¹ and less than or equal to 10 mm⁻¹, and a height of the first microlens protruding from the first surface is greater than or equal to 0.0252 mm and less than or equal to 0.034 mm.

12. The optical lens according to any one of claims 1 to 11, wherein a diameter of an occupied region of the second microlens on the first surface is greater than or equal to 0.05 mm and less than or equal to 0.1 mm, a curvature of an end that is of the second microlens and that is away from the first surface is 0 mm⁻¹, a curvature of an end that is of the second microlens and that is connected to the first surface is greater than or equal to 5 mm⁻¹ and less than or equal to 40 mm⁻¹, and a height of the second microlens protruding from the first surface is greater than or equal to 0.01 mm and less than or equal to 0.0175 mm.

13. The optical lens according to any one of claims 1 to 12, wherein the microlens array is integrally formed with the light-transmitting substrate, and material of the microlens array and the light-transmitting substrate comprises at least one of glass, sapphire, polyamide, polycarbonate, polymeric methyl methacrylate, thermoplastic polyurethanes, and transparent nylon.

14. A photoplethysmograph, comprising:
a cover plate, wherein the cover plate comprises a first light transmissive part and a second light transmissive part;
a light emitter, wherein the light emitter is located on an inner side of the cover plate, and a light emitting surface of the light emitter faces the first light transmissive part;
a light receiver, wherein the light receiver is located on the inner side of the cover plate, and a light incident surface of the light receiver faces the second light transmissive part; and
an optical lens, wherein the optical lens is the optical lens according to any one of claims 1 to 13, the optical lens is disposed on a surface that is of the first light transmissive part and that faces the light emitter, a first surface of the optical lens faces the light emitter, and a second surface faces the first light transmissive part.

15. An electronic device, comprising: a display, a casing, and the photoplethysmograph according to claim 14, wherein
the display is connected to the casing, the casing comprises a back cover, and the back cover is opposite to the display; and
a cover plate of the photoplethysmograph is disposed on the back cover, and a light emitter and a light receiver of the photoplethysmograph are located in the casing.

## Patentansprüche

1. Eine optische Linse (265), umfassend:
ein lichtdurchlässiges Substrat (2651), wobei das lichtdurchlässige Substrat (2651) mit einer ersten Oberfläche (S1) und einer zweiten Oberfläche (S2) versehen ist, die einander gegenüberliegen; und
ein Mikrolinsenarray (2652), wobei das Mikrolinsenarray (2652) auf der ersten Oberfläche (S1) angeordnet ist, das Mikrolinsenarray (2652) umfasst eine erste Mikrolinsenbaugruppe (2652a) und mehrere zweite Mikrolinsenbaugruppenarrays (Ar1), wobei jedes zweite Mikrolinsenbaugruppenarray (Arl) mehrere zweite Mikrolinsenbaugruppen (2652b) umfasst, die in einer Anordnung um die erste Mikrolinsenbaugruppe (2652a) herum angeordnet sind, und wobei die mehreren zweiten Mikrolinsenbaugruppenarrays (Ar1) entlang einer radialen Richtung einer ringförmigen Array-Bahn der mehreren zweiten Mikrolinsenbaugruppen (2652b) nacheinander angeordnet sind, wobei
sowohl die erste Mikrolinsenbaugruppe (2652a) als auch die zweite Mikrolinsenbaugruppe (2652b) eine erste Mikrolinseneinheit (C1) und ein zweites Mikrolinsenarray (Ar2) umfassen, wobei die erste Mikrolinseneinheit (C1) mindestens eine erste Mikrolinse (T1) umfasst, und das zweite Mikrolinsenarray (Ar2) mehrere zweite Mikrolinsen (T2) umfasst, die in einer Anordnung um die erste Mikrolinseneinheit (C1) herum angeordnet sind; und
sowohl die erste Mikrolinse (T1) als auch die zweite Mikrolinse (T2) sind konvexe Kugeln, die von der ersten Oberfläche (S1) in eine Richtung weg von der zweiten Oberfläche (S2) hervorstehen.

2. Die optische Linse nach Anspruch 1, wobei zwei benachbarte zweite Mikrolinsenbaugruppenarrays in Abständen und eng entlang der radialen Richtung der ringförmigen Array-Bahn der mehreren zweiten Mikrolinsenbaugruppen angeordnet sind, und die erste Mikrolinsenbaugruppe und das benachbarte zweite Mikrolinsenbaugruppenarray ebenfalls in Abständen und eng entlang der radialen Richtung der ringförmigen Array-Bahn der mehreren zweiten Mikrolinsenbaugruppen angeordnet sind.

3. Die optische Linse nach Anspruch 1 oder 2, wobei mehrere zweite Mikrolinsenbaugruppen in jedem zweiten Mikrolinsenbaugruppenarray ebenfalls in Abständen und eng entlang einer ringförmigen Array-Bahn der mehreren zweiten Mikrolinsenbaugruppen angeordnet sind.

4. Die optische Linse nach einem der Ansprüche 1 bis 3, wobei das zweite Mikrolinsenarray und die erste Mikrolinseneinheit in Abständen und eng entlang einer radialen Richtung einer ringförmigen Array-Bahn der mehreren zweiten Mikrolinsen angeordnet sind.

5. Die optische Linse nach einem der Ansprüche 1 bis 4, wobei die mehreren zweiten Mikrolinsen im zweiten Mikrolinsenarray ebenfalls in Abständen und eng entlang der ringförmigen Array-Bahn der mehreren zweiten Mikrolinsen angeordnet sind.

6. Die optische Linse gemäß Anspruch 5, wobei die Anzahl der ersten Mikrolinsen in der ersten Mikrolinseneinheit eins beträgt und die Anzahl der zweiten Mikrolinsen im zweiten Mikrolinsenarray sechs beträgt.

7. Die optische Linse gemäß Anspruch 4 oder 5, wobei sich eine Vielzahl von ersten Mikrolinsen in der ersten Mikrolinseneinheit befindet, wobei die Vielzahl der ersten Mikrolinsen in einer Reihe angeordnet ist und die Anordnungsrichtung der Vielzahl der ersten Mikrolinsen in der zweiten Mikrolinsenbaugruppe mit der Verlängerungsrichtung einer ringförmigen Arraybahn der zweiten Mikrolinsenbaugruppe übereinstimmt, in der sich die zweite Mikrolinsenbaugruppe befindet.

8. Die optische Linse gemäß Anspruch 7, wobei in der ersten Mikrolinsenbaugruppe und der zweiten Mikrolinsenbaugruppe die Vielzahl der ersten Mikrolinsen in Abständen und dicht nebeneinander angeordnet sind.

9. Die optische Linse gemäß Anspruch 8, wobei sowohl in der ersten Mikrolinsenbaugruppe als auch in der zweiten Mikrolinsenbaugruppe die Anzahl der ersten Mikrolinsen zwei beträgt und die Anzahl der zweiten Mikrolinsen im zweiten Mikrolinsenarray zwölf beträgt.

10. Die optische Linse gemäß Anspruch 8, wobei sowohl in der ersten Mikrolinsenbaugruppe als auch in der zweiten Mikrolinsenbaugruppe die Anzahl der ersten Mikrolinsen zwei beträgt und die Anzahl der zweiten Mikrolinsen im zweiten Mikrolinsenarray sechs beträgt.

11. Die optische Linse gemäß einem der Ansprüche 1 bis 10, wobei der Durchmesser eines belegten Bereichs der ersten Mikrolinse auf der ersten Oberfläche größer oder gleich 0,1 mm und kleiner oder gleich 0,15 mm ist, wobei die Krümmung eines Endes der ersten Mikrolinse, das von der ersten Oberfläche entfernt ist, 0 mm-1 beträgt, die Krümmung eines Endes der ersten Mikrolinse, das mit der ersten Oberfläche verbunden ist, größer oder gleich 8 mm-1 und kleiner oder gleich 10 mm-1 ist und die Höhe der ersten Mikrolinse, die von der ersten Oberfläche hervorsteht, größer oder gleich 0,0252 mm und kleiner oder gleich 0,034 mm ist.

12. Die optische Linse gemäß einem der Ansprüche 1 bis 11, wobei der Durchmesser eines belegten Bereichs der zweiten Mikrolinse auf der ersten Oberfläche größer oder gleich 0,05 mm und kleiner oder gleich 0,1 mm ist, wobei die Krümmung eines Endes der zweiten Mikrolinse, das von der ersten Oberfläche entfernt ist, 0 mm-1 beträgt, die Krümmung eines Endes der zweiten Mikrolinse, das mit der ersten Oberfläche verbunden ist, größer oder gleich 5 mm-1 und kleiner oder gleich 40 mm-1 ist und die Höhe der zweiten Mikrolinse, die von der ersten Oberfläche hervorsteht, größer oder gleich 0,01 mm und kleiner oder gleich 0,0175 mm ist.

13. Das optische Objektiv gemäß einem der Ansprüche 1 bis 12, wobei das Mikrolinsenarray integral mit dem lichtdurchlässigen Substrat gebildet ist und das Material des Mikrolinsenarrays und des lichtdurchlässigen Substrats mindestens eines der folgenden umfasst: Glas, Saphir, Polyamid, Polycarbonat, polymeres Methylmethacrylat, thermoplastische Polyurethane und transparentes Nylon.

14. Ein Photoplethysmograph, umfassend:
Eine Abdeckplatte, wobei die Abdeckplatte einen ersten lichtdurchlässigen Abschnitt und einen zweiten lichtdurchlässigen Abschnitt umfasst;
Eine Lichtquelle, wobei die Lichtquelle auf der Innenseite der Abdeckplatte angeordnet ist und eine lichtemittierende Oberfläche der Lichtquelle zum ersten lichtdurchlässigen Abschnitt zeigt;
Ein Lichtempfänger, wobei der Lichtempfänger auf der Innenseite der Abdeckplatte angeordnet ist und eine lichtempfangende Oberfläche des Lichtempfängers zum zweiten lichtdurchlässigen Abschnitt zeigt; und
Eine optische Linse, wobei die optische Linse die optische Linse gemäß einem der Ansprüche 1 bis 13 ist, die optische Linse auf einer Oberfläche angeordnet ist, die zum ersten lichtdurchlässigen Abschnitt gehört und zur Lichtquelle zeigt, eine erste Oberfläche der optischen Linse der Lichtquelle zugewandt ist, und eine zweite Oberfläche dem ersten lichtdurchlässigen Abschnitt zugewandt ist.

15. Eine elektronische Vorrichtung, umfassend: ein Display, ein Gehäuse und den Photoplethysmographen gemäß Anspruch 14, wobei
Das Display mit dem Gehäuse verbunden ist, das Gehäuse eine Rückabdeckung umfasst und die Rückabdeckung dem Display gegenüberliegt; und
Eine Abdeckplatte des Photoplethysmographen auf der Rückabdeckung angeordnet ist und eine Lichtquelle sowie ein Lichtempfänger des Photoplethysmographen sich im Gehäuse befinden.

## Revendications

1. Une lentille optique (265), comprenant :
un substrat transmettant la lumière (2651), le substrat transmettant la lumière (2651) étant doté d'une première surface (S1) et d'une seconde surface (S2) opposées ; et
un réseau de microlentilles (2652), le réseau de microlentilles (2652) étant disposé sur la première surface (S1), le réseau de microlentilles (2652) comprenant un premier assemblage de microlentilles (2652a) et une pluralité d'ensembles d'arrays de secondes microlentilles (Ar1), chacun des ensembles d'arrays de secondes microlentilles (Ar1) comprenant une pluralité de secondes assemblages de microlentilles (2652b) disposés en array autour du premier assemblage de microlentilles (2652a), et la pluralité d'ensembles d'arrays de secondes microlentilles (Ar1) étant disposés de façon séquentielle le long de la direction radiale d'une piste d'array annulaire de la pluralité de secondes assemblages de microlentilles (2652b), dans lequel
le premier assemblage de microlentilles (2652a) et le second assemblage de microlentilles (2652b) comprennent tous deux une première unité de microlentille (C1) et un deuxième réseau de microlentilles (Ar2), la première unité de microlentille (C1) comprenant au moins une première microlentille (T1), et le second réseau de microlentilles (Ar2) comprenant une pluralité de secondes microlentilles (T2) disposées en array autour de la première unité de microlentille (C1) ; et
la première microlentille (T1) et la seconde microlentille (T2) sont toutes deux des sphères convexes faisant saillie à partir de la première surface (S1) dans une direction opposée à la seconde surface (S2).

2. La lentille optique selon la revendication 1, dans laquelle deux ensembles d'arrays de secondes microlentilles adjacents sont disposés de manière espacée et rapprochée le long de la direction radiale de la piste d'array annulaire de la pluralité de secondes assemblages de microlentilles, et le premier assemblage de microlentilles et l'ensemble d'array de secondes microlentilles adjacent sont disposés de manière espacée et rapprochée le long de la direction radiale de la piste d'array annulaire de la pluralité de secondes assemblages de microlentilles.

3. La lentille optique selon la revendication 1 ou 2, dans laquelle une pluralité de secondes assemblages de microlentilles dans chaque ensemble d'array de secondes microlentilles sont également disposées de manière espacée et rapprochée le long d'une piste d'array annulaire de la pluralité de secondes assemblages de microlentilles.

4. La lentille optique selon l'une quelconque des revendications 1 à 3, dans laquelle le second réseau de microlentilles et la première unité de microlentille sont disposés de manière espacée et rapprochée le long de la direction radiale d'une piste d'array annulaire de la pluralité de secondes microlentilles.

5. La lentille optique selon l'une quelconque des revendications 1 à 4, dans laquelle la pluralité de secondes microlentilles dans le second réseau de microlentilles sont également disposées de manière espacée et rapprochée le long de la piste d'array annulaire de la pluralité de secondes microlentilles.

6. Lentille optique selon la revendication 5, dans laquelle le nombre de premières microlentilles dans la première unité de microlentilles est égal à un, et le nombre de secondes microlentilles dans le réseau de secondes microlentilles est égal à six.

7. Lentille optique selon la revendication 4 ou 5, dans laquelle il existe une pluralité de premières microlentilles dans la première unité de microlentilles, lesdites premières microlentilles sont disposées en ligne, et la direction d'agencement de la pluralité de premières microlentilles dans le second ensemble de microlentilles est cohérente avec la direction d'extension de la piste en réseau annulaire du second ensemble de microlentilles dans lequel ledit second ensemble est situé.

8. Lentille optique selon la revendication 7, dans laquelle, dans le premier ensemble de microlentilles et le second ensemble de microlentilles, la pluralité de premières microlentilles sont disposées à intervalles rapprochés.

9. Lentille optique selon la revendication 8, dans laquelle, dans le premier ensemble de microlentilles et le second ensemble de microlentilles, le nombre de premières microlentilles est égal à deux, et le nombre de secondes microlentilles dans le réseau de secondes microlentilles est égal à douze.

10. Lentille optique selon la revendication 8, dans laquelle, dans le premier ensemble de microlentilles et le second ensemble de microlentilles, le nombre de premières microlentilles est égal à deux, et le nombre de secondes microlentilles dans le réseau de secondes microlentilles est égal à six.

11. Lentille optique selon l'une quelconque des revendications 1 à 10, dans laquelle le diamètre d'une région occupée par la première microlentille sur la première surface est supérieur ou égal à 0,1 mm et inférieur ou égal à 0,15 mm, la courbure de l'extrémité de la première microlentille opposée à la première surface est de 0 mm-1, la courbure de l'extrémité de la première microlentille connectée à la première surface est supérieure ou égale à 8 mm-1 et inférieure ou égale à 10 mm-1, et la hauteur de la première microlentille dépassant de la première surface est supérieure ou égale à 0,0252 mm et inférieure ou égale à 0,034 mm.

12. Lentille optique selon l'une quelconque des revendications 1 à 11, dans laquelle le diamètre d'une région occupée par la seconde microlentille sur la première surface est supérieur ou égal à 0,05 mm et inférieur ou égal à 0,1 mm, la courbure de l'extrémité de la seconde microlentille opposée à la première surface est de 0 mm-1, la courbure de l'extrémité de la seconde microlentille connectée à la première surface est supérieure ou égale à 5 mm-1 et inférieure ou égale à 40 mm-1, et la hauteur de la seconde microlentille dépassant de la première surface est supérieure ou égale à 0,01 mm et inférieure ou égale à 0,0175 mm.

13. Lentille optique selon l'une quelconque des revendications 1 à 12, dans laquelle le réseau de microlentilles est formé intégralement avec le substrat transmissif de lumière, et le matériau du réseau de microlentilles et du substrat transmissif de lumière comprend au moins un des éléments suivants : verre, saphir, polyamide, polycarbonate, polyméthacrylate de méthyle, polyuréthanes thermoplastiques et nylon transparent.

14. Un photopléthysmographe, comprenant :
une plaque de recouvrement, la plaque de recouvrement comprenant une première partie transmissive à la lumière et une seconde partie transmissive à la lumière ;
un émetteur de lumière, l'émetteur de lumière étant situé sur un côté intérieur de la plaque de recouvrement, et une surface d'émission de lumière de l'émetteur fait face à la première partie transmissive à la lumière ;
un récepteur de lumière, le récepteur de lumière étant situé sur le côté intérieur de la plaque de recouvrement, et une surface d'incidence de lumière du récepteur fait face à la seconde partie transmissive à la lumière ; et
une lentille optique, la lentille optique étant la lentille optique selon l'une quelconque des revendications 1 à 13, la lentille optique étant disposée sur une surface de la première partie transmissive à la lumière et faisant face à l'émetteur de lumière, une première surface de la lentille optique fait face à l'émetteur de lumière, et une seconde surface fait face à la première partie transmissive à la lumière.

15. Un dispositif électronique, comprenant : un écran d'affichage, un boîtier et le photopléthysmographe selon la revendication 14, dans lequel
l'écran d'affichage est connecté au boîtier, le boîtier comprend un couvercle arrière, et le couvercle arrière est opposé à l'écran d'affichage ; et
une plaque de recouvrement du photopléthysmographe est disposée sur le couvercle arrière, et un émetteur de lumière et un récepteur de lumière du photopléthysmographe sont situés dans le boîtier.
